(19) Europäisches Patentamt
European Patent Office
Office européen des brevets

(11) Publication number: **0 125 139**
**A2**

(12) # EUROPEAN PATENT APPLICATION

(21) Application number: 84303090.9

(22) Date of filing: 08.05.84

(51) Int. Cl.³: G 01 N 33/54
C 12 Q 1/68
//C07F17/02, C07D473/08

(30) Priority: 05.05.83 GB 8312263
05.05.83 GB 8312259
05.05.83 GB 8312265
21.09.83 GB 8325316
16.12.83 GB 8333650
16.12.83 GB 8333651
19.01.84 GB 8401399
29.02.84 GB 8405262
29.02.84 GB 8405263

(43) Date of publication of application:
14.11.84 Bulletin 84/46

(84) Designated Contracting States:
BE CH DE FR GB IT LI NL SE

(71) Applicant: Genetics International, Inc.
50 Milk Street Fifteenth Floor
Boston, MA 02109(US)

(72) Inventor: Hill, Hugh Allen Oliver
9 Clover Close
Oxford OX2 9JH(GB)

(74) Representative: Clifford, Frederick Alan et al,
MARKS & CLERK 57/60 Lincoln's Inn Fields
London WC2A 3LS(GB)

(54) **Assay techniques utilising specific binding agents.**

(57) This specification discloses assay techniques and especially those for the detection measuring and monitoring of complex, usually biologically-arising, molecules or part-molecular structure often in complicated admixture with other similar molecules. The techniques of the invention utilise specific binding agents, i.e.g complex molecular species which have a specific reaction, usually based on electrostatic or hydrophobic forces, with one or more active sites on a given type of molecule only.

One limb of this invention is concerned with immunoassay techniques for ligands and analytes to be carries out in vivo or in vitro. It should be noted that these ligands include species which are not only *per se* antigenic, but may be made so by crosslinking them to a suitable carrier.

Another limb of the present invention is concerned with; firstly techniques for investigaging DNA or RNA sequencing, and secondly with other types of reactions in which specific, non-immunological binding occurs. The specification further discloses equipment or materials useful in the above techniques, either as such or possibly as usable coordinated kits of parts.

The specification particularly discloses, an assay system comprising

(a) a ligand-antiligand pair or analyte-specific binding agent pair capable of specifically binding together, ($T_4A$)

(b) a mediator (F) enzyme (GOD) and corresponding substrate (GLUCOSE) triplet, whereby conversion of the substrate into a product by the enzyme generates electrical charge which is transferred *via* the mediator to an electrode;

wherein at least some of one of the pair and at least some of one of the triplet are chemically cross-linked (GOD-$T_4$, so as to inhibit at least a part of the said charge transfer when the ligand and antiligand pair or analyte-specific binding agent pair are bound together and,

wherein the addition of either one of the said pair or one of the said triplet changes the rate of charge transfer to the electrode.

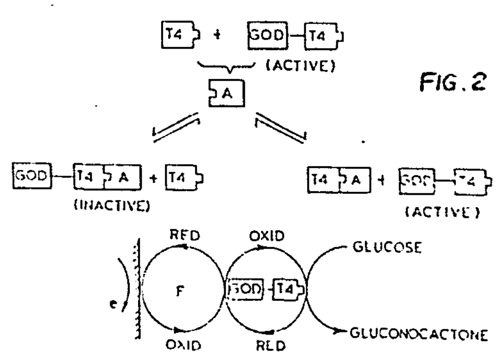


FIG. 2

EP 0 125 139 A2

M&C FOLIO: 46068X WANGDOC: 0104r

Title: ASSAY TECHNIQUES UTILISING SPECIFIC BINDING AGENTS

This invention generally relates to assay techniques and especially to the detection measuring and monitoring of complex, usually biologically-arising, molecules or part-molecular structures often in complicated admixture with other similar molecules. The techniques of this invention utilise specific binding agents, i.e. complex molecular species which have a specific reaction, usually based on electrostatic or hydrophobic forces, with one or more active sites on a given type of molecule only.

One limb of this invention is concerned with immunoassay techniques to be carried out in vivo or in vitro i.e. those techniques in which large antibody or antigen molecules, naturally present in or artificially induced to appear in a body fluid such as plasma, serum, whole blood, urine, interstitial fluid, cerebrospinal fluid or synovial fluid (or made as monoclonal antibodies) are reacted with usually smaller, specifically-reacting ligand molecules. It should be noted that these ligands include species which are not per se antigenic, but may be made so by crosslinking them to a suitable carrier.

Another limb of the present invention is concerned with; firstly techniques for investigating DNA or RNA sequencing i.e. techniques in which part of a molecular strand is reacted with its specific and complementary counterpart,and secondly with other types of reactions in which specific, non-immunological binding occurs, such as in the reaction of; enzymes with inhibitors or cofactors; receptors with hormones, vitamins, toxins or growth-factors; glycoconjugates with lectins; nucleic acids with proteins; macromolecules containing aromatic groups with dyes; and metal-interacting macromolecules with metal ions.

For convenience of description the two limbs of the invention will be jointly referred to as specific binding reactions between ligands and antiligands.

The invention further relates to equipment or materials useful in the above techniques, either as such or possibly as usable coordinated kits of parts.

Known immunoassay techniques are closely related in that they are based on the competitive displacement of labeled ligand from an antibody complex by the unlabeled substances in the assay sample,the amount of labeled ligand displaced being proportional to the amount of unlabeled ligand in the assay sample. For example, in

the assay of drugs such methods include:

a) Radioimmunoassay (RIA); in which technique the drug or other species to be assayed is labeled with a radioactive isotope such as $^{3}H$, $^{14}C$, $^{131}I$, $^{75}Se$, $^{35}S$ and $^{125}I$ and quantitated by scintillation counters. RIA is a so-called heterogeneous immunoassay since it requires a separation step to remove the displaced labeled drug from the antibody bound drug prior to quantitation. RIA is a powerful technique with sensitivities of the order of $10^{-8}$ to $10^{-10}$M. Among the drugs to which this technique has been applied are analgesics such as aspirin, antibiotics such as adriamycin and gentamycin, anticonvulsants such as phenytoin, antineoplasmic agents such as methotrexate, etc. Among the disadvantages of this method are the short shelf-life and biological hazards associated with the use of radioactive materials.

b) Spin Immunoassay; in which the label is a free radical of a stable type, whose unpaired electron is detectable by its characteristic spin resonance. In this case the ESR spectrum of free spin labeled ligands is quite different (sharp and high peaks) from that of bound spin-labeled ligands (broad and low peaks). It is therefore unnecessary to separate bound and free ligands and this type of immunoassay is said to be a homogeneous

immunoassay. The sensitivity of this method is in the range $10^{-4}$ to $10^{-5}$ Molar at maximum. A significant limitation of this method is the highly expensive and specialized nature of the instrumentation and staff training required. In an example of this method, morphine was labeled with a nitroxide spin label: in solution this compound gives three sharp ESR lines, whereas when bound a broadened single peak (which may be difficult to detect above noise) is seen.

c) Homogeneous Enzyme Immunoassay (also known as Enzyme Linked Immunoassay - ELISA); in which an enzyme is conjugated with the drug to be assayed in such a way that it does not alter its enzyme activity. In view of the comparatively large size of enzymes this is sometimes difficult to achieve. However, when the drug-enzyme conjugate is bound to the antibody specific to the drug, structural (conformational) changes take place which then reduce the enzyme activity, or the active site of the enzyme is blocked by steric hindrance.If free drug is present in the sample it will bind to the antibody thus releasing the enzyme-drug conjugate. Thus, enzyme activity is proportional to the quantity of free drug present in the sample. The immunoassay is reduced to the assay of the enzyme activity and therefore, separation of free and bound drug is not required. Furthermore, the label used in this assay

affords a degree of amplification as one molecule of unlabeled drug can liberate an enzyme molecule which may catalyse the conversion of many molecules of the substrate for the enzyme into a detectable species. These assays unfortunately often require a colorimetric determination of enzyme activity. Homogeneous enzyme immunoassays have been developed for drugs of abuse such as heroin, methadone, cocaine, THC, LSD, STP and PCP, antiasthmatic drugs such as theophylline, cardioactive drugs such as lidocaine, for the thyroid hormones such as thyroxine and for drugs used in theraputic control such as Ethosuximide. Hetergoeneous enzymelinked immunoassays are also widely used.

d) Fluorescence Excitation Transfer immunoassay; this approach employs two labels, and is based on the phenomenon of fluorescence excitation transfer, which permits rapid detection of low concentration of drugs. In one variant of this method, the drug is labled with a fluorescer such as fluorescein II and the antibody is labeled with an acceptor (quencher) such as rhodamine III. If the average distance between the quencher and the fluorescer within the (drug)(labled-antibody) complex is close enough to permit dipole-dipole coupling and energy transfer, then the formation of a complex leads to the quenching of fluorescence. The addition of unlabeled drug to this assay mixture reduces the amount of quenching.

Unfortunately this method also requires expensive equipment and highly trained staff.

Known DNA (RNA) probe techiques share a similarity in that the DNA (RNA) polymer is not readily detectable by its inherent biochemical activity. It is therefore necessary to mark the polymer with some signal-producing chemical or biochemical species, such methods include:-

a) Avidin-Biotin Reaction; this technology relies on the affinity of the egg-white glycoprotein avidin for biotin. Biotin (Vitamin W) can be covalently linked to the nucleotide residues which comprise the monomeric subunits of the DNA polymer. The modified subunits can still undergo the classical binding reaction between complementary strands of double-stranded DNA, and thus can be incorporated into synthetic DNA probes. To detect the presence of such probes which have formed short double-stranded regions after exposure to complementary sample DNA, the unbound probe must first be separated from the sample DNA/bound probe complex. This is normally done by performing the binding reaction in conditions under which the sample DNA is immobilized on a substrate and washing, although centrifugation may perform the same function. The bound probe is detected by the addition of avidin to which either a fluorescent-marker-labeled antibody or an enzyme has been attached.

One problem with the above method is that small oligonucleotide probes (20 nucleotides) contain only a small number of biotinylated sites, limiting the amount of avidin which can be bound. Attempts have been made, with some success, to add long "tails" of up to several thousand bases to the probe DNA, in which case only the tail need be labeled. The method can detect up to a resolution of $10^{-13}$g of DNA, or about $10^5$ copies of a single gene. Although originally the marker on the avidin was horse radish peroxidase (a rather short-lived enzyme) the method has now been extended to include alkaline phosphatase. Unfortunately the method is generally difficult to establish for a new diagnostic scheme as either the biotin-linked probe DNA is difficult to prepare or the labeled tail interferes with sensitivity.

b) Radio-isotopic methods: these were the original methods of detecting specific DNA sequences. A DNA probe is constructed from $^3H$, $^{14}C$ or $^{32}P$ loaded nucleotides, and the binding of the probe to a target sequence is detected by measuring the emission of the DNA duplex formed on hybridisection, by autoradiography. In this method it is common practise to digest the target DNA with restriction endonucleases and separate the fragments on an agarose gel by electrophoresis before "blotting" onto a nitrocellulose

filter which binds the target DNA and holds it in place during exposure to the radioactive probe. Unfortunately, it may take several weeks to obtain an autoradiograph of a particular nitrocellulose plot, and the reagents employed in this method are again subject to decay and potentially hazardous.

c) <u>Other methods</u>; include indirect detection of the particular DNA sequences of interest by the use of "restriction enzyme fragment length polymorphism" (RELP). Such a method can be employed for the prenatal detection of disease by the investigation of as little as 100ug of fetal DNA taken from the trophoblast, which is of fetal origin. Chemoluminescent labeling has also been employed, but with a mixed degree of success.

One aim of the DNA probe technology and the enzyme detection/assay technology so far developed, has been to detect inbuilt errors of metabolism which lead to a variety of "genetic diseases" and inheritable disorders. Among such disorders are: familial Goiter (iodotyrosine dehalogenase defective), Maple syrup urine disease ($\alpha$-keto decarboxylase defective), Xanthinuria (Xanthine oxidase defective) and Methemoglobinemia (Methemoglobin reductase defective). A full list of 3500 conditions due to defective genes can be found in McKusick's "Mendelian Inheritance in Man".

As can be seen from the above, however, the methods of detailed metabolic assay presently available have certain disadvantages in that they require some or all of the following; short lifetime reagents (either radioactive, air-sensitive or light-sensitive), highly trained staff or (for autoradiography, ESR measurements or low-level light detection) expensive equipment.

Known methods of assay for binding reactions other than antigen/antibody and DNA/complementary DNA are generally similar to the above methods and suffer from similar problems.

The present invention sets out to overcome the above disadvantages by utilising simple electrochemical measurement techniques, and is related to that invention described in copending European Patent Application 82305597 .

It is an object of the present invention to provide a method for the detection, measurement, or monitoring of specific binding agents which is free from the above defects.

It is a further object of the present invention to provide apparatus and reagents to perform such methods.

The prior applications referred to above all concern the direct measurement by a suitable electrode of the progress of the catalytic reaction of an enzyme on its specific substrate, utilising a mediator compound to transfer charge to the electrode.

The present invention is based on a realisation that, other things being equal, a measurable difference is achieved (a) if the effective level of mediator is altered (b) if the effective level of enzyme is altered, (c) if both are altered (d) if the effective surface area of the electrode changes; and is further based on the realisation that such alterations can be brought about by specific binding reactions between a ligand and an antiligand, in such ways that the measurable effect can be related back to the occurrence or extent of ligand/antiligand binding.

In one aspect therefore the invention consists in a method of assay in which (a) at least one specific binding reaction stage between reactive species comprising a ligand and an antiligand is carried out in association with an enzyme/mediator system electrochemically linked to a level of a substrate to which the enzyme is catalytically active so that the binding reaction affects the electrochemical availability of at least one of the components of said

system and (b) the effect on the said electrochemical availability is detected or measured to give a signal from which the presence or amount of the said reactive species can be established.

The procedures of the present invention involve the use of ligand and antiligand, e.g. antigen/antibody or DNA target sequence/DNA probe sequence; substrate; enzyme specific to the substrate; and mediator compound. In the procedures the specific binding reaction can take place wholly in solution i.e. be homogeneous, or can take place on a solid surface.e.g. can possibly be heterogeneous. Since the solid surfaces utilised in these procedures can include the wall of a containing vessel, or an electrode surface, and since such an electrode itself can be a simple carbon electrode or can be of gold or other noble metal (for example) or can be more or less complex with coatings of one or more of the various components, especially mediator, antibody or antiligand and enzyme but possibly substrate,there are many variant methods by which the invention defined above can be embodied. These methods, and their usefulness and advantages, will be set out, and exemplified in detail below.

Conversely certain features of procedure and materials can be utilised in all aspects of the invention; more

especially the nature of the enzyme/substrate on the one hand, and the mediator on the other.

The enzyme/substrate pair are present merely as an indicator of the presence or extent of the specific binding reaction. Thus, in theory, they can be any such pair which is extraneous to the environment of the reaction, i.e. whose electrochemical or other action will not be confused with those of components naturally present in the total test mixture. Of course, if the method chosen depends wholly or partly on reducing the enzyme level or electrochemical availability of enzyme (rather than wholly on mediator level) then the enzyme chosen should be such that the specific binding reaction affects that availability.

Enzyme/substrate pairs whose electrochemical behaviour in association with mediator compounds have been studied by the Applicants include the following:-

| Enzyme | Substrate |
|---|---|
| Flavo-proteins | |
| Pyruvate Oxidase | Pyruvate |
| L-Amino Acid Oxidase | L-Amino Acids |
| Aldehyde Oxidase | Aldehydes |
| Xanthine Oxidase | Xanthines |
| Glucose Oxidase | Glucose |

| Enzyme | Substrate |
|---|---|
| **Flavo-proteins** | |
| Glycollate Oxidase | Glycollate |
| Sarcosine Oxidase | Sarcosine |
| Lactate Oxidase | Lactate |
| Glutathione Reductase | NAD(P)H |
| Lipoamide Dehydrogenase | NADH |
| **PQQ Enzymes** | |
| Glucose Dehydrogenase | Glucose |
| Methanol Dehydrogenase | Methanol and other Alkanols |
| Methylamine Dehydrogenase | Methylamine |
| **Haem-Containing Enzymes** | |
| Lactate Dehydrogenase (Yeast Cytochrome B2) | Lactate |
| Horse-radish Peroxidase | Hydrogen Peroxide |
| Yeast Cytochrome C Peroxidase | Hydrogen Peroxide |
| **Metalloflavoproteins** | |
| Carbon monoxide Oxidoreductase | Carbon Monoxide |
| **Cuproproteins** | |
| Galactose Oxidase | Galactose |

Of these, it is clearly advantageous to utilise those enzyme/substrate pairs whose behaviour is established in most detail and which give good, preferably linear, response over the expected measurement range. The glucose enzymes have been carefully studied for test purposes relating to glucose sensing in vivo (e.g. for diabetic subjects). Enzymes such as the NADPH-independent glucose dehydrogenases e.g. as obtained from acinetobacter calcoaceticus have been found valuable for this purpose, and can be used in the present invention. Glucose oxidase undergoes reactions which are well known and thus form a useful marker for the extent or occurrence of a specific binding reaction in accordance with the present invention. It is also stable in storage, industrially available and of low cost

While there is a some preference for glucose oxidase, the other enzymes listed above can be used with advantage in certain variant forms of the invention.In particular, the reaction speed of glucose dehydrogenase can be of value.

The mediator compounds suggested for use in our prior Patent Applications listed above include polyviologens, chloranil, bromanil, etc, but are most preferably the compounds generally known as "metallocenes" and especially those which comprise at least two organic

rings, each of which comprises a charge-conjugated system and a metal atom in electron-sharing contact with each of the said rings.

Ferrocenes (bis-cyclopentadienyl iron and its derivatives) fall within the last above named group and have advantages over other mediators used with enzyme/substrate reactions for charge-transfer purposes.

The unique structure and properties of ferrocene (bis $7^5$ cyclopentadienyliron: $Fecp_2$) and its derivatives has resulted in a considerable amount of theoretical and

experimental study.First synthesised in 1951, ferrocene was the earliest example of the now well-known metallocene compounds.

Whilst ferrocenes had been found of limited value in spectrophotometric assays as a result of their poor solubility in aqueous solution and low extinction coefficients, they have been found to be more suited to a bio-electrochemical system. Ferrocenes have: (a) a wide range of redox potentials accessible through substitution of the cyclopentadienyl rings which can be functionalised; (b) electrochemically reversible one-electron redox properties; (c) the pH-independent redox potential and the slow autoxidation of the reduced form.

Within this general class of ferrocenes, i.e. the monomeric or polymeric derivatives substituted around one or both rings, we have found certain individual ferrocenes such as are listed below:

| Ferrocene derivative | Eo | Solubility | E |
|---|---|---|---|
| 1,1'-dimethyl | 100 | I,D | - |
| acetic acid | 124 | S | 370 |
| hydroxyethyl | 161 | S | - |
| ferrocene | 165 | I,D | 335 |
| 1,1'bis(hydroxymethyl)- | 224 | S | 385 |

| Ferrocene derivative | Eo | Solubility | E |
|---|---|---|---|
| monocarboxylic acid | 275 | S | 420 |
| 1,1'-dicarboxylic acid | 385 | S | - |
| chloro | 345 | I,D | - |
| methyl trimethylamino | 400 | S | - |

(where S indicates water solubility; I,D means respectively insoluble and detergent solubilised in 3% Tween-20; $E^o$ is in mV vs a standard calomel electrode, E is measured in $cm^{-1}M^{-1}$) to have advantages.

The $E^o$ values of various ferrocenes in phosphate buffer at pH 7.0 given in the above table, span a range of potentials, $E^o$ = 100 to 400mV vs SCE. The trend in $E^o$ values is in agreement with that expected on the basis of substituent effects. In general electron-donating groups stabilize the positive charge and hence promote oxidation more than electron withdrawing groups.

It is moreover to be noted that, as described more fully below, a chemical linkage between either the ligand or enzyme and the mediator can be present in certain specialised embodiments. This is of particular use in the monitoring of theraputic drugs, where it is envisaged that the ferrocene or other mediator may be chemically bound to a ligand. It is envisaged, for

example, that the following drugs may be combined with a mediator to configure an assay system capable of determining whether or not the concentration of the drug is within the preferred theraputic range:

Phenobarbital

Phenytoin

Procainamide

Theophylline.

Other drugs can also be used.

Ferrocene-modified electrodes can be prepared by a number of different techniques. The simplest procedure is to dope hydrophobic derivatives e.g. ferrocene, vinylferrocene and 1,1'-dimethylferrocene on to a platinum or graphite surface by evaporation from a solution in an organic solvent. Alternatively, substituted derivatives can be covalently attached to hydroxyl functions on either carbon or platinum as below:

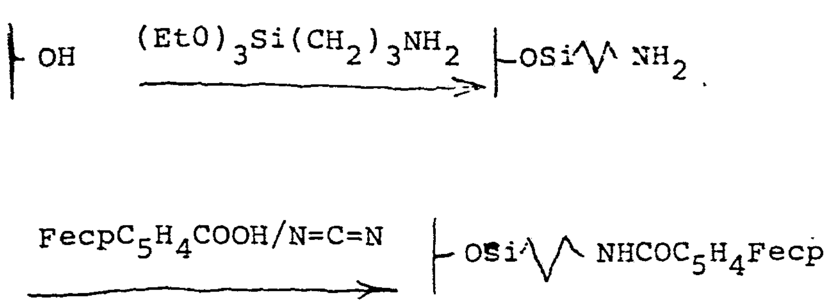


A special case of such modification is to incorporate one or more thiol groups whereby ferrocene can link

directly to gold electrodes, as described in more detail below.

Electrode surfaces may also be coated with a functionalised polymer, polytyramine for example, to which substituted ferrocenes are covalently attached, as below

$$\vdash NH_2 \xrightarrow{cpFeC_5H_4CHO} \vdash N{=}CC_5H_4Fecp$$

$$\xrightarrow{NaBH_4} \vdash NHCH_2C_5H_4Fecp$$

Alternatively, vinylferrocene can be polymerised to give poly(vinylferrocene), $(CH_2\text{-}CHC_5H_4Fecp)_n$, which can be coated on to an electrode either by solvent evaporation or by electro-deposition

The ferrocene carboxylic acids have been found to have an optimal combination of practical properties for general use in this invention, giving the possibilities of chemical linkages, as discussed below, through the carboxyl group(s) to the ligand, antiligand and/or enzyme.

Certain procedures within the present invention have shown themselves to be especially useful as practical assay techniques. Among these we have discovered

I. A homogeneous immunoassay technique for detecting or measuring the amount of a desired ligand e.g. antigen or antibody in a mixture.

II. A homogeneous immunoassay technique for detecting or measuring the amount of an analyte chemically linkable to the enzyme and also capable of a specific binding reaction to a ligand.

III. A homogeneous nucleic-acid probe technique wherein specific binding to a complementary sequence affects electrochemical availability of one or more species in the mixture.

IV. A heterogeneous immunoassay technique related to but improved over the known colorimetric ELISA technique.

V. An immunoassay technique in which the surface of a suitable electrode is covered by or altered by the specific binding reaction so as to affect transfer of charge on to the electrode.

Each of the above will now be described further;

I. Homogeneous immunoassay technique for a ligand

In one aspect this consists in an assay system comprising;

a) a ligand-antiligand pair capable of specifically binding together,

b) a mediator, enzyme and corresponding substrate triplet, whereby conversion of the substrate into a product by the enzyme generates electrical charge which is transferred *via* the mediator to an electrode;

wherein at least some of one of the pair and at least some of one of the triplet are chemically cross-linked, so as to inhibit at least a part of the said charge transfer when the ligand and antiligand pair are bound together, and,

wherein the addition of either one of the said pair or one of the said triplet changes the rate of charge transfer to the electrode.

In one embodiment of this form the invention consists in a method as described above in which an unknown amount of a ligand species to be determined is subjected to a specific binding reaction with a known amount, known to be in excess, of a species antiligand therefor itself chemically linked with the said mediator compound which antiligand species in its unbound state only is available for charge transfer from the enzyme/substrate

reaction, whereby the subsequent electrochemical activity of the excess unbound mediator/antiligand species provides a measure of the amount of ligand species.

In a second embodiment of this form the invention consists in a method as described above in which an unknown amount of a ligand species to be determined is subjected to a specific binding reaction with a known amount, known to be in excess, of a species antiligand therefor itself chemically linked with the enzyme which antiligand species in its unbound state only is available for an enzyme/substrate reaction, whereby the subsequent electrochemical activity of a mediator in transferring charge from unbound enzyme/antiligand species provides a measure of the amount of ligand species.

The term "ligand species" is utilised to cover (a) an initial antigen or antibody i.e. as naturally occurring in or artificially stimulated to occur in, a human or animal subject, and presented either as a serum, whole blood, urine, interstitial fluid, cerebrospinal fluid or synovial fluid for assay or in wholly or partially purified form, or as a monoclonal antibody and (b) a partly reacted form or complementary derivative material from such an antigen/antibody e.g. as formed with a

known amount, known to be in excess, of its own antiligand. For reasons of stability or availabilty of materials it may be preferred to work "stepwise" on the raw assay material until the unknown quantity is measurable in a convenient desired species.

Thus, by way of example the invention is this form further consists in a method of homogeneous enzyme immunoassay which comprises:

(a) (i) mixing a sample containing ligand to be assayed with a known amount, known to be in excess, of an antiligand whereby some ligand-binding sites on the antiligand are left free;

(ii) mixing with the resultant mixture a known amount, known to be in excess, of the ligand chemically linked with a mediator compound whereby all of the previously free ligand-binding sites are occupied and some ligand-linked mediator remains electrochemically available;

(b) mixing an enzyme and a substrate therefor in a liquid mixture;

(c) contacting the enzyme/substrate mixture with the mixture from stage (a); and

(d) contacting the resulting mixture with a sensor electrode;

whereby the charge transferred to the electrode is dependent upon the amount of available unbound ligand-linked mediator and thus permits derivation of the amount of the original ligand.

The man skilled in the art will appreciate that, while features (i) and (ii) are necessarily consecutive, there is some permitted variability in the order of the other steps. In particular, the enzyme, substrate and electrode can be made up and the stage (a) mixture added; or the stage (a) mixture can be formed within the liquid system of a pre-existing enzyme substrate mixture, and so on.

Moreover, other stepwise procedures could be envisaged, provided that they reduce the unknown quantity to a form bindable to the mediator-linked material for eventual measurement. Again the man skilled in the art will not always expect the mathematical derivation to be simple subtraction: if one ligand/antiligand has several sites for binding, some distribution of binding can be expected and/or some equilibrium binding of different species. These features can be measured and accounted for in the eventual mathematical derivation.

In this aspect of the invention a valuable enzyme-substrate system is glucose/glucose oxidase.

II. Homogeneous enzyme immunoassay for ligand analyte

In a first embodiment of this form the invention consists in the method described generally above in which (i) (a) an unknown amount of ligand species X to be assayed and (b) an amount of a species X-E, in which X is chemically linked with an enzyme E without destroying the enzyme activity thereof, are mixed in solution (ii) the mixture is contacted with a substrate S for the enzyme and a mediator compound M in the presence of a sensor electrode to which a measured charge is thereby transferred in dependence on the enzymatically catalysed reaction (iii) antiligand A is contacted with the solution to set up competitive equilibrium binding reactions of the general nature of reactions I and II.

$$A + X + X = E \rightleftharpoons \text{I} \quad A - X = E + X$$
$$\rightleftharpoons \text{II} \quad A - X + X = E$$

whereby part of the enzymatically active species X = E is converted to the enzymatically inactive species A-X=E thereby altering the extent of reaction and the

measured charge at the electrode and (iv) there is derived from the decrease or rate of decrease in charge a measure of the concentration of analyte.

As a modification of the above, the mediator M can be chemically linked to the ligand analyte X and can encounter the enzyme E and substrate S at step (ii), setting up a directly analogous competitive reaction in step (iii) i.e. one in which M is substituted for E throughout.

It is possible though less preferable to operate starting from the complex A-X=E (or A - X =M) and relying only on the slow displacement reaction:-

A-X=E + X $\rightarrow$ A + X + X =E $\rightleftharpoons$ A-X + X=E, e.g. if a "dry strip" test for level of analyte X is to be developed. This, in one form this comprises a method of immunoassay for a species X, which comprises:

(a) covalently linking an analyte X and an enzyme E to give an enzymatically active species X=E;

(b) mixing with the covalently linked species X-E a suitable reactive ligand A to form an enzymatically inactive species A-X=E with its specific binding sites fully occupied;

(c) contacting the species A-X=E with the species X to be assayed, so as to set up a competitive specific binding reaction with species X and achieve the equilibrium

$$A\text{-}X{=}E + X \rightleftharpoons A + X + X{=}E \rightleftharpoons A - X + X = E$$

so that the total amount of species X=E is a measure of the amount of species X to be assayed; and

(d) contacting the mixture with a substrate for X=E, and transferring charge from the consequent enzymatically catalysed reaction to a sensor electrode by means of a mediator compound to give a measure of the total amount of enzymatically active species X=E present, from which the amount of species X can be derived.

It will be found preferable to link the enzyme and analyte (X=E) close to the enzyme active site so that the ligand A effectively blocks, or causes conformational change at, the site.Alternatively, it may be desirable to link the ligand analyte X to the prosthetic group of the enzyme.

Once again, the exact sequence of steps used, and the presence of additional steps can be varied by a man skilled in the art. Moreover, it may be possible to operate with the A-X=E complex located at the electrode itself, whereby contact with analyte X takes some of the

ligand off the electrode and allows enzymatic activity to take place with the substrate and species X-E at the electrode surface.

In this general form of the invention the amount of enzymatically active species is measured. It therefore lends itself to the further optional feature mentioned above and described in more detail below, in which the enzyme and mediator are themselves chemically linked.

III. Homogeneous assay using nucleic acid probe

In this form the invention consists in a method as described generally above in which at least one of the mediator and enzyme is chemically linked to a nucleic acid probe sequence whereby specific binding of the probe sequence to the target sequence in a single-strand nucleic acid material to be investigated affects the electrochemical availability of the chemically linked species as detected by a sensor electrode in presence of the enzyme substrate, whereby the presence of the target sequence can be detected.

The nucleic acid sequence can be RNA e.g. messenger RNA but is usually DNA.

Expressed otherwise, this form of the invention consists

in a method of detecting a target sequence in a nucleic acid material which comprises:

(a) providing a single strand nucleic acid material to be investigated for a given target sequence;

(b)selecting a probe material with a sequence of nucleic acids complementary to the target sequence;

(c) choosing a procedure from among (i) chemically linking the probe with an enzyme and adding the enzyme-linked probe to a solution containing both a substrate for the enzyme and a mediator,

(ii) chemically linking the probe with a mediator and adding the mediator-linked probe to a solution containing both a substrate and an enzyme for the said substrate;

(iii) chemically linking the probe with a mediator/ enzyme combination and adding the so-modified probe to a solution containing a substrate for the enzyme;

(d) contacting the solution containing the chemically linked probe sequence with a sensor electrode whereby charge is transferred by the mediator to the electrode from the enzyme-catalysed substrate reaction; and

(e) contacting the solution with the single stranded material,

whereby alteration in the amount of charge transferred is an indication of a specific binding reaction between the probe and target affecting the availability of enzyme, mediator or combination.

The probe material can be a naturally occuring DNA fragment or a synthetically produced material.

Alterations in the sequence of steps can be readily envisaged. Also the sensor electrode itself can include the mediator or the enzyme, although generally it is preferred for the probe sequence and the target sequence both to be present in solution.

The mediator can be linked indirectly to the probe sequence by a linker group, and a material reactive to the linker groups can be present on the electrode. In this case the whole complex is present on the electrode; when a target sequence is present, and binds to the probe sequence, an alteration in electrode current is produced.

IV. Heterogeneous enzyme immunoassay derived from the ELISA technique

In this form the invention consists in the method generally described above, which comprises immobilising a ligand to be assayed on a suitable surface; thereafter carrying out a specific binding reaction with excess of a suitable antiligand itself chemically linked to an enzyme, prior to removing the excess and adding a substrate for the immobilised enzyme; and contacting with a mediator and a sensor electrode, so that the charge transferred to the electrode is proportional to the amount of enzyme present.

In a preferred embodiment it envisages a method of heterogeneous enzyme-linked immunoassay comprising:
(a) immobilising at a suitable surface a ligand to be assayed by e.g. by binding it to an antiligand species already attached to the surface.

(b) contacting with the ligand excess of an enzymatically active species consisting of an antiligand chemically linked with an enzyme to bring about a specific binding reaction with the immobilised ligand and give an immobilised enzymatically active species in an amount corresponding to that of the ligand to be assayed;

(c) removing excess non-immobilised chemically-linked enzyme/antiligand, and

(d) contacting the immobilised enzyme with a substrate therefor and with a charge-transferring mediator compound whereby an electrode in contact therewith signals a charge corresponding to the amount of immobilised enzyme, from which the original amount of ligand to be assayed may be derived.

The above steps may be modified in their order, and the mediator may be present in the solution or on the electrode.

V Enzyme immunoassay at the electrode surface

In this form the invention consists in the method generally described above in which the specific binding reaction takes place at the surface of a sensor electrode to block or alter the characteristics of the surface at least in part whereby the existence or amount of a decrease in detected electrical charge is a measure of the existence or extent of the specific binding reaction.

One preferred embodiment consists in a method of immunoassay comprising the steps of:

(a) taking a liquid medium containing a ligand to be assayed;

b) adding thereto a known amount of a substrate capable of undergoing enzyme-catalysed reaction with an enzyme;

(c) contacting the liquid medium with a sensor electrode comprising at its surface a combination of (i) antiligand capable of undergoing a specific binding reaction with the ligand to be assayed (ii) the said enzyme and (iii) a mediator compound to transfer charge from the enzyme to the electrode when the enzyme is catalytically active, so as to provide a signal; and

(d) comparing the signal with a signal received in the absence of the ligand, and deriving the amount of ligand present as a function of blockage or conformational change at the electrode surface caused by the specific binding reaction.

The ligand, immunologically reactive with the antiligand, can be any antigen arising in biochemical or medical testing as exemplified above. Their general nature, as large protein-like molecules, appears to have the effect of blocking off part of the electrode surface when they react with the antiligand thereon. This causes a drop in current, the observation or extent of which indicates or measure the presence or level of ligand. It is also possible to link a smaller molecule or hapten e.g. nitroglycerine with a larger protein

molecule such as Bovine Serum Albumen; inject this combination into an animal subject, to generate antiligand sensitive to nitroglycerine; and combine this antiligand on to the electrode. In such a case the small bound nitroglycerine molecules probably would not block the electrode surface, but (especially in higher concentrations of antibody and/or nitroglycerine) may lead to conformational changes in the antiligand and thus changes in signal.

The substrate material can in theory be any of the substrates listed in our various Patent Applications set forth above or listed herein, since any underlying enzyme/substrate reaction is of general applicability with such electrodes. However, it will be found convenient to use a simple, readily available, well-documented substrate and we prefer to use glucose for this purpose.

The enzyme present on the electrode can similarly be any enzyme, especially those flavoprotein and quinoprotein enzymes listed above. However, since the preferred substrate is glucose the preferred enzymes are glucose oxidase or glucose dehydrogenase e.g. as obtained from acinetobacter calcoaceticus. The latter is preferable to the former as having a hundred fold higher turnover number.

In the above discussion of the invention there has been mentioned the optional use in some cases of enzyme/mediator linked compounds.

Usually in this form of practice of the present invention more than one of the "sandwich-type" units e.g. ferrocene will be present in the enzyme, up to a concentration at which distortion of the molecule and loss of enzyme activity is observed. By way of example, a glucose oxidase with about 8-12 ferrocene units is still enzymatically active and could be bound to carbon electrodes by methods known per se e.g. use of carbodiimide bonding. The modified enzyme is not only enzymatically active but also electrochemically active.

While the above description is generally concerned with methods of assay it will be apparent to the person skilled in the art that modified sensor electrodes and coordinated kits of parts including such electrodes, also form part of the invention. More especially, strip test electrodes, so configured as to contain all of the necessary components in accordance with the invention, whereby they can merely be dipped into a test liquid, are envisaged.

The invention will be further described by way of example and with reference to the accompanying drawings

wherein all Figs 1, 2, 3a - 3c, 4 and 5, show diagrammatically assay procedures in accordance with the invention and Fig. 6 compares graphically the antigenicity of a drug and a drug/mediator conjugate.

EXAMPLE 1

Assay employing Glucose Oxidase and Ferrocene

Turning to figure 1 the serum of a patient containing antigens (1) to be assayed is mixed with a solution of antibodies (2) of known strength in buffer. The antigen may, for example, be a drug. Antigens (1) in the serum bind to the solvated antibodies at specific antigen-binding sites (3), to form antigen/antibody complexes (4) Not all of the possible antigen-binding sites on the antibody are occupied and therefore a number of free binding sites (5) remain. The number of free sites is a function of the number of antigens (1) in the serum.

A mediator-linked antigen (6) is added, in excess, to the mixture resulting from step 1. In this example the mediator (7) is a ferrocene derivative which is chemically linked to the antigen (8), but it is envisaged that other metallocenes could be substituted. The ferrocene-linked antigen occupies the free sites (5)

of the antibody (2) by virtue of an antigen/antibody binding reaction. However, the ferrocene-linked antigen (6) is present in excess over the number of free sites and therefore some of the ferrocene-linked antigen remains in solution (9).

Glucose and glucose oxidase (abbr. GOD) are added in excess to the mixture resulting and the current established by the redox action of GOD on glucose is measured with an electrode (E). Only the ferrocene-linked antigen complexes (9) which are not bound to the antibodies (2) are able to electrochemically couple GOD to the electrode (11). The ferrocene-linked antigen complexes which are bound to the antibodies are prevented from coupling GOD to the electrode because of steric hindrance from the much larger antibody (2).

By measuring the electrochemical effect of the mediator present after the addition of the enzyme (GOD) and its substrate it is therefore possible to calculate how much of the known quantity of mediator (6) is bound to the antibodies (2) and therefore to determine how much free antigen (1) must have been present in the original sample.

The electrode (E) may be a made of gold, carbon,

platinum or any suitable material.

The addition of GOD and glucose may be replaced by the addition of any other enzyme/substrate pair providing that the particular mediator selected is capable of coupling the enzyme/substrate reaction to the electrode. Neither the enzyme nor the substrate (both present in excess)i.e. in this example neither GOD nor glucose, are limiting factors; the limiting factor is the amount of mediator which is freely diffusing when the free sites have been occupied.

EXAMPLE 2.

Assay employing Glucose- oxidase- labeled T4

A scheme illustrating this assay forms figure 2. All assays were performed at ambient temperatures (18-25°C) with excess of substrate (100 mM), in 50 mM Tris/HCl buffer pH 7.5. No corrections for non-specific binding were made. The solutions were all degassed and ferrocene monocarboxylic acid was used as a mediator. The electrode used was a pyrolytic graphite electrode polished between runs using a slurry of 0.3 alumina in $H_2O$. No components were immobilized and a three electrode cell was used consisting of

(i) Pyrolytic graphite electrode

(ii) Platinum counter electrode.

(iii) Calomel reference electrode.

Total sample volume was 1 ml.

Glucose oxidase- labelled $T_4$ Thyroxine and antibodies to $T_4$ thyroxine were supplied by Corning Glass Inc. (Medfield, Mass, U.S.A.).

To the sample solution, consisting of Tris/HCL pH 7.5 buffer was added ferrocene monocarboxylic acid. A current of 1.66 uA was noted. Upon addition of 100 mM glucose in excess this current was observed to fall to 1.463 uA.

When 50 uls of GOD labelled $T_4$ were added the observed current rose to 2.10 uA, due to the catalytic activity of the glucose oxidase upon available glucose. Electrons removed from the glucose oxidase were transferred via the mediator to the electrode.

Addition of antibodies to the GOD-labelled-$T_4$ reduced this current to 1.59 µA.

It is clear from these results that addition of antibodies will inhibit the activity of the GOD-labelled $T_4$, causing a detectable and measurable change in the electrode current which can be employed to give a quallitative and/or quantitative determination of the analyte. This is illustrated in figure 2, which demonstrates the competitive reaction between the enzyme linked analyte and the free analyte for the binding sites of the antibody.

Only when the GOD-labelled $T_4$ is not bound to the antibody is the enzyme capable of oxidising glucose. The resulting electrons are shuttled to the electrode by the mediator compound and the mediator is regenerated at the electrode surface.

EXAMPLE 3

Techniques involving nucleic acid probes

Example 3a--ENZYME ATTACHED TO DNA PROBE

(I)In Figure 3a, glucose oxidase(GOD) catalyses the conversion of glucose to gluconic acid and liberates electrons, e, which reduce a ferrocene mediator F from its oxidised to its reduced condition. Reduced ferrocene ion is oxidised at the electrode

and the current passed is measured and is proportional to the amount of glucose present.

Linked to the GOD by any suitable method is a DNA fragment D which may be either derived from a naturally occuring DNA sequence or may be synthetic.

(II) In the presence of an excess of the glucose a steady-state current is obtained. A sample of DNA which is to be assayed for a particular target sequence complementary to the sequence of the DNA fragment D is converted to single strands by any suitable method, and then added to the reaction mixture. If a target sequence complementary to the DNA fragment D is present in the mixture, it will bind to the fragment D and inhibit the enzymatic reaction of the enzyme with the glucose . Consequently the throughput of glucose S to glucose acid will be reduced and the coupled reduction of ferrocene will be diminished. The change in the rate of reduction of ferrocene is reflected in a reduction of the current at the electrode . The change in current is proportional to the amount of the fragmented DNA D (which is now bound to target DNA) and hence to the amount of target DNA present.

Example 3b --MEDIATOR ATTACHED TO DNA PROBE

(I)In example 3b, a redox-active substituted ferrocene F is attached directly(Fig. 3b) to the fragmented DNA which is to be used as a probe. The formation of mediator-linked DNA-probe does not impede either the amperometric response of the mediator F nor the binding interaction of the mediator-DNA probe with the complementary target sequence contained in the DNA which is being assayed.

(II)The amperometric response caused by the addition of the mediator-DNA probe to the assay mixture is measured. If genetic material in a single-stranded form to which the probe is complementary is present, the probe binds to the complementary sequence in the sample DNA. This greatly reduces or completely inhibits the amperometric response, that is, the complex of the mediator-DNA probe and the target DNA is not amperometrically active. The reduction in the initial amperometric response is in direct proportion to the amount of mediator-DNA probe/target DNA complex formed and hence to the amount of genetic material containing a sequence complementary to the known sequence of the mediator-DNA probe.

In this example, the enzymatic activity is unchanged but

the extent to which it is transferred to the electrode is altered.

Example 3c--MEDIATOR-AND- LINKER ATTACHED TO DNA PROBE

(I) In this example (as shown in Figure 3c) the ferrocene-DNA probe also contains one or more linker groups L, (biotin may for example be used). An electrode on whose surface is present an electrochemically active material R, that recognises the linker group L( for example avidin labelled with ferrocene) is then immersed in the reaction mixture. The current is measured on applying a potential

(II) The mediator-linker-DNA probe is now treated with the mixture of the single-stranded genetic material and the mediator-linker-DNA probe binds to any complementary sequence present. The original current is reduced on the binding of the electrochemically active material R to the mediator-linker-DNA probe /target-DNA complex. The reduction of the current is again in proportion to the amount of the added single-stranded sequence with a target sequence complementary to the known sequence of the DNA-probe.

Although the present invention has in this example been described in terms of DNA (Deoxyribonucleic acid) it is

equally applicable to forms of RNA such as messenger RNA.

EXAMPLE 4

Heterogeneous (two step) assay procedure

As shown in Figure 4, an antibody is placed in a polyester or polysytrene container (11), at which time some of the antibody (12) binds to the walls of the container. The container may be treated to enhance the binding of the antibody, and/or to prevent subsequent non-specific binding of antibody (12) as described below. This treatment to avoid or reduce non-specific binding can include the coating of the container (11) with Bovine Serum Albumen .

An antigen sample is added at 12d, and binds to the antibody itself linked to the walls of the container.

The antibody- linked enzyme, in this example Glucose oxidase (15) linked to further antibody (12) is added, and the antigen/antibody binding reaction proceeds.To ensure binding, the antibodies may be different and bind to different portions of the antigen 12a.

The container is washed to remove excess antigen-enzyme complex, and a solution containing a soluble ferrocene

(16) is added together with an aliquot of glucose substrate.

A reference electrode (17), such as a calomel, silver-silver chloride, gold, platinum or any other suitable noble metal electrode is placed in the solution together with an electron transfer electrode (8) which may or may not have an external coating of ferrocene.

The current produced at the electrode (8) is read via line (19).

The current is proportional to the activity of glucose oxidase 15 and therefore bears a stoichiometric relationship to the antibody glucose combination and hence to the sample antigen 12a to which this binds. If desired, the mediator could be antibody-linked, and the enzyme could be added with the substrate. Other variations are also possible.

<u>EXAMPLE 5</u>

<u>Blocking of mediator/enzyme coupling at the electrode surface</u>

The schematic form of this example is shown in figure 5.

(I). In this example a ligand is being assayed for. An antiligand 20 capable of binding specifically to the ligand is immobilized on the electrode surface E. In the absence of the ligand 21 the mediator22 in solution is free to transfer charge from the enzyme ENZ to the electrode surface when the enzyme is catalytically active on substrate S.

(II). However, when the ligand 21 is present in the solution, the ligand appears to have the effect of blocking off part of the electrode surface when bound with the antiligand thereon, thus causing a drop in current, the observation or extent of which indicates or measures the presence or level of ligand.This assay system is of value when the ligand is large e.g. an immunoglobin such as IgE.

EXAMPLE 6

Preparation of a modified enzyme

Ferrocenemonocarboxylic acid was coupled to glucose oxidase using isobutylchloroformate. The terminal amine of the protein was involved in the reaction with a carbonic anhydride of ferrocene.

The reaction was performed as follows; to a stirred cooled (-8$^{o}$C) solution of Ferrocenemonocarbocylic acid

(1mM 235mg in 3ml of dry tetrahydrofuran), isobutylchloroformate (0.13ml) and triethylamine (0.14ml) were added with constant stirring. Care must be taken at this stage to keep the reaction water-free and prevent hydrolysis of the anhydride product. A drying-tube was attached to the apparatus which was otherwise sealed, and had been previously purged with Argon.

The mixture was stirred for thirty minutes at -8°C, and then allowed to warm to room temperature and stirred for another hour. The resulting carbonic anhydride of Ferrocene was added dropwise to a cooled (2°C) solution of glucose oxidase (150mg in 50ml of 0.1M $NaHCO_3$ solution). As the Ferrocene was added the pH was maintained at 8 with 0.1M $NaHCO_3$. The reaction mixture was stirred at 4°C for 24 hours and then centrifuged. This removed a large proportion of unreacted Ferrocene and any precipitated enzyme. The protein was then exhaustively dialysed against pH 8.5 borate buffer (0.2M boric acid, 0.05M borax, adjusted to pH with the latter).

Characterisation of modified enzyme

Approximately 10% of the amino-acid residues on glucose oxidase are available for modification, and hence one

would expect many Ferrocenes to be coupled to each enzyme molecule. To calculate how many the following procedure was adopted.

The iron content of the modified enzyme was determined by atomic adsorption. To determine the protein content, a spectrophotometric assay technique was used. The assay is based on the shift of absorbance from 465nm to 595nm of an organic dye. Coomassie Blue, on binding to the protein at acidic pH.

A 20% aqueous solution of the dye was filtered. A standard solution (1.4mg/ml) of unmodified glucose oxidase was prepared and from this concentrations of glucose oxidase were mixed with the Coomassie Blue solution. Optical densities at 595nm were then meassured at each concentration of enzyme, and a standard curve obtained for the unmodified enzyme (Fig. 4.2).

The protein concentration of the modified enzyme can thus be estimated by finding the optical density of a sample and comparing it with the standard curve. In the sample prepared the protein concentration was found to be 11.04μM. The iron concentration was 87.5μM.

One can therefore deduce that a mean of eight Ferrocenes have been coupled to each moleculer of glucose oxidase.

Electrochemistry of modified enzyme.

From the enzyme preparation above, an 11uM solution of enzyme in borate buffer is obtained. To investigate the electrochemistry of the modified enzyme, cyclic voltammograms were obtained, using the conventional three-electrode cell. Experiments were performed as follows; the cell was filled with 800uL of modified enzyme (11uM). The reference arm had been previously filled with pH 8 borate buffer.

Typical diffusion-control kinetics show that the ferrocene molecule (linked to the enzyme through an amide) acts as a reversible, one electron mediator and shows Type I kinetics.

In the absence of glucose the enzyme exists as its oxidsed form, enzyme-FAD, and no catalytic reaction is seen; however, one addition of -D-glucose the enzyme exists as enzyme-$FADH_2$ and the catalytic reaction can take place between this reduced form and a ferricinium ion at oxidising protentials.

The second-order rate-constant, k, for the reaction between ferrocene and the reduced form of glucose oxidase was calculated as $3.5 \times 10^5 m^{-1} s^{-1}$.When this value is compared to the one obtained for solution

kinetics of the same system (1.15 x 10 $^{5}m^{-1}s^{-1}$) it is seen that modification causes an increase in the rate of reaction.

The enzymes in the table above have also been coupled electrochemically *via* ferrocene monocarbocylic acid:

Each of these enxymes was applied by Sigma Chemical Co. (St. Louis) and used without further purification.

In general the technology exemplified can be applied to any ligand for which there is a "specific binding partner", which will bind specifically to that ligand. Examples of this interaction are:

| LIGAND | SPECIFIC BINDING ANTILIGAND |
| --- | --- |
| antigen | antibody |
| hapten | antibody raised to hapten conjugate |

| LIGAND | SPECIFIC BINDING ANTILIGAND |
|---|---|
| DNA probe or fragment | antibody to polypeptide chain |
| protein or fragments of proteins | antibody to protein fragment. |

The ligand may be (a) an immunologically active protein or polypetide chain (b) a hapten between 100-2,000 molecular weight. In the latter case antibodies may be raised to the hapten by conjugating the hapten to a protein (such as Bovine serum albumin) to render it immunogenic (c) a carbohydrate or other organic molecule which can be rendered immunogenic.

Examples of ligands include:

| | | | |
|---|---|---|---|
| HORMONES | eg | somatotropin | placental lactogen |
| | | thyrotropin | tissue hormones |
| | | insulin | follicle stimulating hormones |
| | | glucagon | |
| | | gonadotropin | |
| | | leutinizing hormone | |

Examples

| | |
|---|---|
| VIRUSES | retroviruses |
| | herpes viruses |
| | hepatitis viruses |
| ENZYMES | blood serum enzymes (amalyase, cholinesterase and others) |
| | cytochromes |
| | prostatic acid phosphatase |
| | monoamine oxidase |

BACTERIA AND FRAGMENTS THEREOF INCLUDING CELL SURFACE MARKERS AND CELL SURFACE POLYSACCHARIDES.

DNA FRAGMENTS

IMMUNOGLOBULINS IgG, IgM, IgA, IgE, IgD and their respective fragments $F_{ab}$ and $F_c$.

BLOOD CLOTTING FACTORS

Examples of hapten Ligands include:

VITAMINS A, B, C, D, E, K, folic acid

DRUGS (a) aminoglycosides eg amikacin, gentamicin, netil micin, tobramycin, sisomicin, kanamycin.

(b) nucleotides eg. FAD, NAD, NADP

(c) steroids eg. cortisol, testosterone

(e) others phenobarbital,
lidocain, amphetamines, catecholamines,
caffeine, digoxin, quinidine
disophyramide,
theophylline, cannabinoids, opiates,
barbiturates, benzodiazepines,
methadon

(f) anti-epileptic drugs, phenytoin,
primidon, phenobarbital, carbamazepine.

(g) anti-neoplastic drugs
methotrexate, mitomycin c
bleomycin, ifosfamide, cyclophosphamid
cisplatinum drugs
vinblastine
vincristine

As also described above, a number of assay schemes can be developed for this technology, both heterogeneous, and homogeneous

These include:-

(i) Ligand bound to ferrocene.All components freely diffusing.

(ii) Ligand bound to ferrocene. Ferrocene functionalised and bound covalently to the electrode surface. Examples of groups used to functionalise ferrocene include $-(CH_2)_n NH_2$ or $-(CH_2)_nCOOH$ or others.

(iii) As (ii) above, but with glucose oxidase adsorbed, cross linked or immobilised on to the electrode surface.

(iv) Ferrocene functionalised and attached to GOD to form a GOD-ferrocene-ligand complex.

(v) Ferrocene functionalised and attached to GOD. Ligand functionalised and attached to GOD separately. The ferrocene may, in turn be attached to the electrode surface if desired.

(vi) Ferrocene free, ligand attached to GOD.

(vii).Ferrocene bound to FAD or prosthetic group of enzyme and also bound to ligand, to form a FAD -Ferrocene-ligand complex The FAD-ferrocene-ligand complex would only couple to the apoenzyme when the

ligand is free of its specific binding partner. The competitive assay to set up this would be:

(GOD (apoenzyme))-X-Ab+Ag $\rightleftharpoons$ AbAg + (GOD apoenzyme)-X

in each case X is FAD-Ferrocene-ligand complex.

Various electrochemical techniques may be used in determining the electrochemical change in the system e.g. differential pulse voltammetry, cyclic voltammetry, or square wave voltammetry. In order to minimise response times kinetic, rather than end point, measurements may be desirable.

It is additionally proposed, in accordance with the present invention that among the mediators should figure the thiol or like sulphur derivatives of ferrocene, whereby the mediator can link directly to a gold or like noble metal electrode.

The thiol group can be directly or indirectly attached to one ring of the ferrocene structure, e.g. by a lower alkyl group containing 1 to 6 carbon atoms. The simple thiol (ferrocene) - SH can be used, prepared as in J. Chem. Soc. 692 (1958) Knox and Pauson. We have also established that of the alkyl thiols ferrocenyl thiobutane is valuable i.e. (ferrocene)-$C_4H_8$-SH. Other more complex thiol-like compounds are possible

e.g. 1,2,3-trithia-(3)-ferrocenophane in which the two rings are linked by a chain of sulphur atoms (a mixture of substances with different numbers of chain sulphur atoms is possible).

The gold electrode can be prepared for repeated use e.g. by dipping into solutions of such compounds, so as to link the mediator ferrocene structure to the conductive metal.

Examples of the production of such materials are as follows:-

EXAMPLE 7

1,2,3-trithia-(3)-ferroceneophane - J. Organometallic Chem. 1971, 27 241)

The literature procedure was followed, but no product was obviously evident from the sublimation of the crude mixture. The sublimed material that had the most obvious (i.e. smelliest) potential was chromatographed on silica (30 cm x 2 cm column) with hexane as eluant to give three products.

1. No sulphur on analysis.

2. C:43.72,H:2.83,S:33.05,$C_{10}H_8FeS_3$ requires C:42.98,S:34.42. Yield 0.45g.

3. 011g of complex molecule, not examined beyond the mass spec. which indicated it was not however a ferrocenophane with a simple number of sulphur atoms.

EXAMPLE 8.

Ferrocene thiopentane (ferrocenyl thiobutane)

1. Ferrocenoyl butyric acid (J.Am. Chem Soc. 1957,79,3420)

$F_c$-CO-$CH_2$-$CH_2$-$CH_2$ COOH

Prepared by the lit. method

2.Ferrocenyl butyric acid

$F_c$-$CH_2$-$CH_2$-$CH_2$-$CH_2$-COOH

Prepared by Clemmenson reduction (zinc/mercury and hydrochlic acid)

3. Ferrocenyl butanol

$F_c$-$CH_2$-$CH_2$-$CH_2$-$CH_2$ $CH_2$ OH

Acid (2) (12g) was dissolved in ether (distilled from sodium/potassium) and treated with lithium aluminium hydride (1.27g) in a nitrogen atmosphere. When reaction was complete the excess lithium aluminium hydride was destroyed using ethyl acetate and then water. The organic phases were separated and the aqueous phase washed with ether (2 x 20 ml). The organic phases were combined and dried ($MgSO_4$ and after filtration the solvent was removed on the rotary evaporator.The red oil resulting had two components. Column chromatography(30 cm x 2 cm) on silica eluted with 1:1 ether:hexane gave the alcohol and an ester, $F_c-CH_2-CH_2-CH_2-CH_2-COOCH_3$

4. Ferrocenyl thiobutane

Compound (3) (400mg) was dissolved in pyridine (10 ml, dried over sodium hydroxide) and cooled in an ice bath. Tosyl chloride (1g) was added and the solution stirred until clear, then left for 24 h at $4^oC$. The mixture, containing solid pyridine hydrochloride, was tipped into ice/water and the tosylate precipated out. This was filtered at the water pump to give a yellow solid. A dried portion of this gave the characteristic tosylate i.r. spectrum. The remainder (0.65g, but still damp) was dissolved in ether: methanol (1:1) and sodium hydrosulphide x $H_2O$ (1.6g) was added while stirring,

the mixture being maintained at 5°C. After 30 min, the ice bath was removed and the mixture allowed to warm to room temperature. After 3 h f.l.c. (silica, 1:1 $Et_2O$:hexane) indicated that reaction was complete. The mixture was reduced to dryness on the rotary evaporator and then dissolved in the minimum of $Et_2O$/hexane (1:11) and chromatographed on silica (60-120 mesh, 25 x 2 cm column, eluted with $Et_2O$/hexane (1.:11). The thiol runs very quickly and was collected in approximately 150 ml. Yield 200 mg of $F_c-CH_2-CH_2-CH_2-CH_2-CH_2SH$.

Example 7

Principle of the Electrochemical Enzyme Immunoassay

The principle of this enzyme linked immunoassay is shown schematically below.

$$FeD + D + Ab \rightleftharpoons AbD + FeD$$

$$\rightleftharpoons AbFeD + D$$

where D = drug

Ab = antibody

FeD = ferrocene drug conjugate

Ferrocene is conjugated to the therapeutic drug of interest. In the absence of its antibody the ferrocene

drug conjugate is free to mediate between glucose oxidase and the electrode. Excess glucose is used so that the enzyme is not substrate limited.

In the presence of its antibody the ferrocene-drug conjugate is bound to the antibody and the ferrocene is no longer free to mediate between the enzyme and the electrode. If, however, the unmodified drug is present in the sample, then it will be able to compete with the ferrocene-drug conjugate for its antibody - thus preventing the binding of the ferrocene conjugate, and leaving it free to mediate between glucose oxidase and the electrode.

An ideal ferrocene labelled therapeutic drug of interest should not change the binding characteristics observed from the natural drug for its antibody, i.e. the antibody should bind the labelled drug as well as the unlabelled drug.

Although the presence of the ferrocene-labelled drugs can be detected directly using electrochemical techniques, using an enzyme coupled system provides a 'built in' means of amplification which will allow very low concentrations to be measured easily.

The following information is offered by way of illustration and not by way of limitation.

(a) Preparation of ferroceneacetonitrile. (Compound I)

A mixture of dimethylaminomethyl ferrocene methiodide (9g) and potassium cyanide (10g) were dissolved in 100ml of water and refluxed for 2 hours. The mixture was then cooled and extracted with ether (3 x 150ml) and the combined extracts washed out with water (6 x 100ml). After drying the ether phase with sodium sulphate, the solution was filtered and the solvent removed in vacuo, to yield a yellow crystalline powder, see J. Org. Chem. 23 (1958) 653.

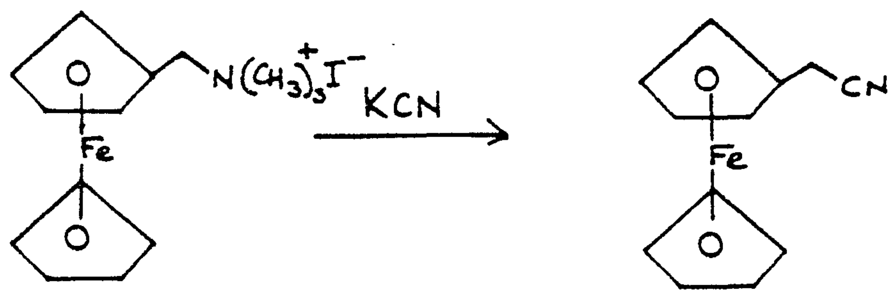


The product had characteristic i.r. absorbances at 2240 $cm^{-1}$, (-CN stretch), 1002 $cm^{-1}$ ferrocene (C-H) bend and 1108 $cm^{-1}$ antisymmetric ring pulsation. Its melting point was in the range 77-78°C.

(b) Preparation of Ferrocene acetic acid. (Compound II)

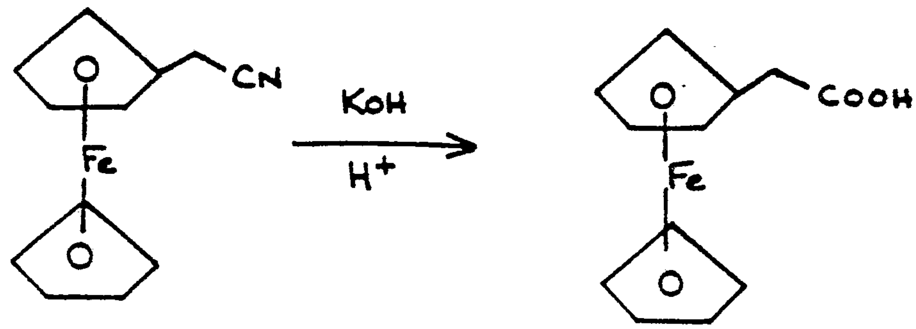


The nitrile (6.20g) was dissolved in ethanol (100ml) and potassium hydroxide (18g) in water (150ml) added and the solution refluxed for 18 hours then cooled. The bulk of the solvent was removed on the rotary evaporator to a final volume of 100ml. This solution was extracted with ether (3 x 150ml) and the aqueous phase then filtered and acidified (to pH 3.0 using orthophosphoric acid, 85%). The yellow precipitate was filtered and dried (phosphoric oxide, desiccator) to give a yellow powder, 4.54g.

Analysis required C: 59.05% H: 4.96%

obtained C: 58.76% H: 4.99%

m.p. 153 -155°C (lit. 152 - 156°)

(c) Preparation of 1,2,3,6,7-pentahydro-1,3-dimethyl-2,6-dioxopurine-8-methyl ferrocene. (Compound III)

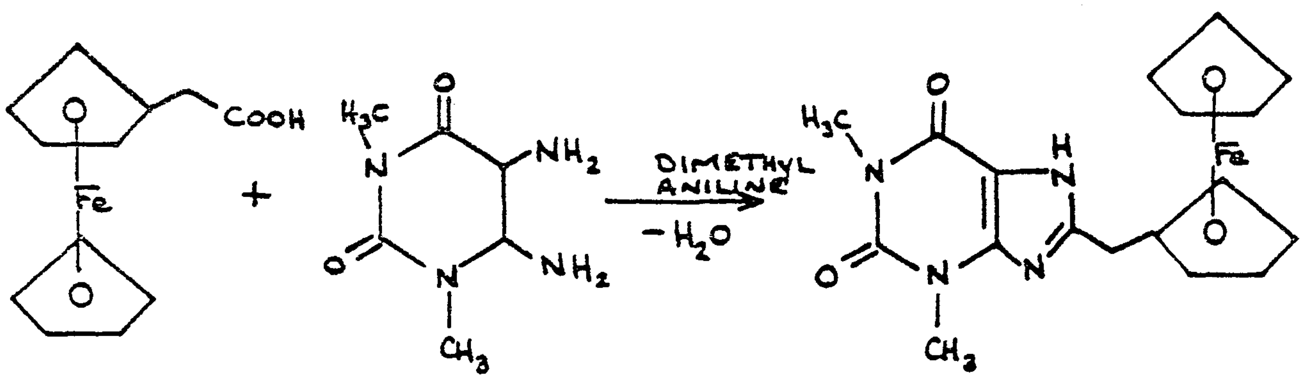


Ferrocene acetic acid (5.1g) and 5,6-diamino-1,3-dimethyluracil hydrate (3.57g) were refluxed under nitrogen in dimethyl aniline (80ml) using a Dean-Stark apparatus. After 18 hours the solution was allowed to cool and aqueous sodium hydroxide solution (8%, 36ml) was added and the solution steam distilled. When complete the pot contents were filtered and then acidified using acetic acid to pH 4.5. The precipitate formed was filtered under suction and dried over phosphorous pentoxide in the vacuum desiccator.

Yield 1.67g, brown powder. m.p. 380°C.

Mass Spec. gives Mol.wt. = 378

micro analysis calculated for $C_{18}H_{18}N_4O_2Fe$

required C:57.14%, H:4.76%, N:14.81%

obtained C:58.00%, H:5.15%, N:13.80%

i.r. has peaks in the 700-800 $cm^{-1}$ region and 1600-1750 $cm^{-1}$ region characteristic of theophylline, and at 1000 $cm^{-1}$ and 1100 $cm^{-1}$ characteristic of a ferrocene derivative substituted in one ring.

From the mass spec. data it would seem this material is very involatile as at $10^{-8}$mm Hg pressure a current of 0.6 amp. was needed to volatilise the sample.

(d) Preparation of ferrocene ethylamine (Compound IV)

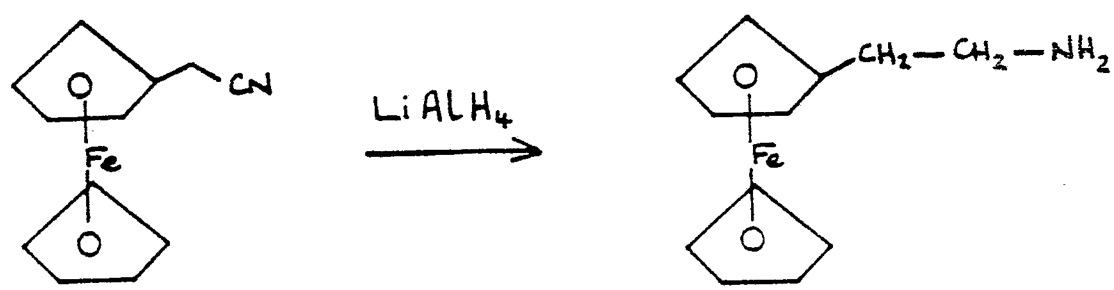


0.85g $LiAlH_4$ was gently refluxed in 40ml of ether for 1 hour. Ferrocenylacetonitrile (2.1g) dissolved in 20ml of ether, was added at a rate that caused reflux to occur while maintaining $N_2$ pressure. Reflux was continued for 2 hours, then the mixture cooled and 2ml of water was added, followed by 1ml of 10% NaOH, and finally 5ml of water. The ether phase was then decanted from the solids, and the latter rinsed with 3 x 10ml of

ether. The combined organic phases were treated with HCl gas to give yellow salt of Ferrocene ethylamine hydrochloride which was separated by decantation under $N_2$. The solid (still soaked with ether) was added to 2M NaOH and the mixture extracted with ether. The ether layer was then dried over $MgSO_4$ and the solvent evaporated <u>in vacuo</u> to yield 1.2g of dark brown oil.

(e) Preparation of 1,2,3,6,7 pentahydro-1,3-dimethyl,-2,6-dioxopurine-8-butanoic acid. (Compound V)

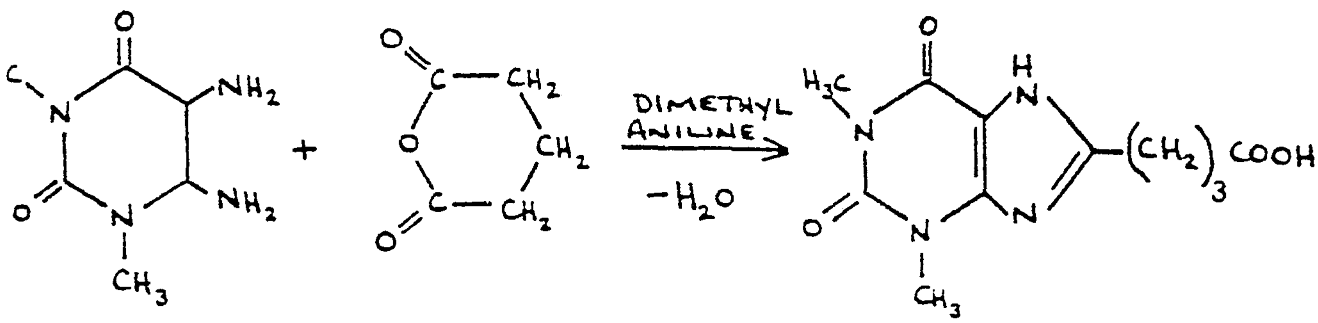


4,5-Diamino-1,3-dimethylpyrimidine-2,5-dione (1g, 5.9g mmole) and glutaric anhydride (1.34g, 11.3mmole) were refluxed in 10ml of N,N-dimethylaniline under $N_2$ for 2.5 hours using a Dean-Stark trap. 5ml solvent was then added and refluxing continued for another 0.5 hours. The reaction mixture was cooled and filtered. The solid material was washed with benzene and

recrystallised from water yielding 600mg of white crystals; m.p: 238 - 240°C; Molecular weight from mass spec. 266

$C_{11} H_{14} N_4 O_4$ Mwt. 266

Micro analysis obtained: C:50.04%; H:5.14%; N:21.12%

Required C:49.62%; H:5.26%; N:21.05%

(f) Preparation of 1,2,3,6,7 pentahydro-1,3-dimethyl-2,6 dioxopurine-8-(N[2- ethyl ferrocenyl] butanamide) (Compound VI)

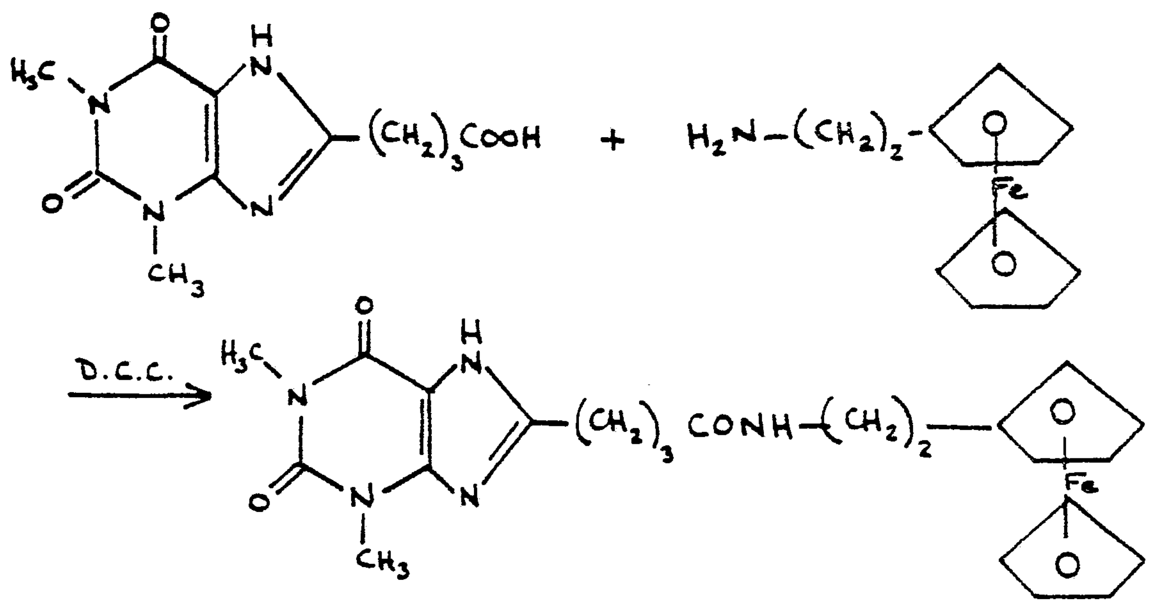

Compound V (133mg, 0.5mmole) and compound IV (115mg, 0.5mmole) were dissolved in 5ml of N,N-dimethylformamide. Dicyclohexylcarbodiimide (120mg, 0.6mmole) was added to the stirred solution at room temperature. Stirring was continued for 4 hours. By that the time reaction was complete as shown by t.l.c. The plate was run using a solvent system of cyclohexane-ethyl acetate-methanol 4:2:1. The reaction mixture was poured into 50ml of ether. The precipitated material was filtered off, and washed well with ether to yield 220mg brownish powder; m.p. 216 - 220°C;

Molecular weight from Mass spec. 477

$C_{23} H_{27} N_5 O_3 Fe$ Mwt. 477

Micro analysis obtained: C:56.80%; H:5.42%; N:14.23%;
required: C:57.86%; H:5.66%; N:14.67%.

The i.r. spectrum has peaks characteristic of theophylline between 700 $cm^{-1}$ and 800 $cm^{-1}$, and also between 1600 $cm^{-1}$ and 1750 $cm^{-1}$ and signals due to a ferrocene component substituted in one ring at 1000 $cm^{-1}$ and 1100 $cm^{-1}$.

(g) Antigenicity of the Conjugates

The ferrocene theophylline conjugates III and VI were assayed to determine their antigenic character, using the Immunotech-double antibody enzyme immunoassay kit. This is a heterogenous enzyme immunoassay method that is based on the principle of competitive binding, where the theophylline ferrocene conjugate or a standard in a sample competes with a theophylline enzyme conjugate for a specific antibody. The separation step is achieved using double antibody immunoprecipitation. Enzyme activity is determined in the pellet and is inversely proportional to the theophylline concentration.

Standard curves were obtained for theophylline in the therapeutic range of interest of 0-40ug/ml.

Standard curves are normally presented as plots of either absorbance at 400nm or the absorbance of the sample expressed as a percentage of the blank versus the log of the concentration. However, a much more informative method is to perform a logit transformation of the data where:

$$\text{logit } \% A/A_o = \ln \frac{\% A/A_o}{(100 - \% A/A_o)}$$

Logit $\%A/A_o$ is then plotted versus the natural log of the concentration to yield a straight line. In order to examine the cross reactivity of a compound, their logit transformations should yield parallel straight lines.

Data are presented in the following tables 7A and 7B and Figure 6 for the standard curve for theophylline, and also for compound III. It was apparent that compound III was more antigenic towards the theophylline antibodies than theophylline itself. Comparison of the results for $\%A/A_o = 50$ (i.e. logit $\%A/A_o = 0$) show that compound III is 7.6 times more antigenic than theophylline.

Similar experiments on compound VI showed it to be approximately 400 times more antigenic than theophylline (data not presented).

TABLE 7A

Data for Antigenicity of Theophylline and the theophylline-ferrocene conjugate (Compound III)

| THEOPHYLLINE ($\mu g\ ml^{-1}$) | Corrected $\%A/A_o$ | logit b |
|---|---|---|
| 0 | - | - |
| 2.5 | 88 | 1.99 |
| 5.0 | 81 | 1.45 |
| 10.0 | 67 | 0.71 |
| 20.0 | 49 | -0.04 |
| 40.00 | 35.5 | -0.60 |

logit b = ln(b/100-b)  b = $\%A/A_o$

r = -0.998  slope = -0.96

TABLE 7B

Theophylline Ferrocene Conjugate (Compound III)

| $\mu g\ ml^{-1}$ (theophylline) | $\%A/A_o$ | logit b |
|---|---|---|
| 0.25 | 84 | 1.66 |
| 0.50 | 78 | 1.22 |
| 1.00 | 69.6 | 0.83 |
| 2.50 | 50.1 | 0.004 |
| 5.00 | 33.2 | -0.70 |
| 7.50 | 25.6 | -1.07 |
| 10.00 | 18.0 | -1.52 |
| 15.00 | 15.9 | -1.66 |
| 20.00 | 12.4 | -1.96 |

r = -0.997 slope = -0.95

(h) Electrochemistry of Ferrocene-Theophylline Conjugates

Reagents

Glucose oxidase (EC 1.1.3.4) was supplied by Boehringer Mannheim. D-Glucose (AnalaR) was from BDH. All solutions were prepared from Aristar grade reagents (BDH) in high purity water (Millipore); supporting electrolyte was 0.1 M $K_2HPO_4$ adjusted to the required pH with $HClO_4$.

Apparatus

D.C. cyclic voltammetry experiments were performed with a two compartment cell that had a working volume of 0.5 ml. In addition to the 4mm gold disk working electrode, the cell contained a 1 $cm^2$ platinum gauze counter-electrode and a saturated calomel electrode as reference. All potentials are referred to the saturated calomel electrode (S.C.E.).

For d.c. cyclic voltammetry, an Oxford Electrodes potentiostat was used with a Bryans X-Y 26000 A3 chart recorder.

Procedure

Both compound III and VI were insoluble in aqueous solvents therefore the required quantities were dissolved in a small amount of dimethylformamide (DMF) and this solution added to buffer solutions to give a final DMF concentration of 10% (v:v). In this manner it was possible to keep compounds III and VI in aqueous solution for the electrochemical experiments.

Results

Under the experimental conditions used in this study and over the complete range of potential scanned (-100 to 500 mV) vs S.C.E. and range of potential scan rates ( = 2 - 50mV $s^{-1}$), both compound III and VI showed voltammograms consistent with a reversible one-electron redox agent at a gold electrode.
($E_p$ = 60mV : $i_p$/ $^{1/2}$ = constant).

Compound III, $E_{1/2}$=195mV, Compound VI, $E_{1/2}$=150mV

Since compound VI proved to be extremely antigenic towards the theophylline antibodies (approximately 400 times that of theophylline) it was decided to study compound III in more detail, as this compound is only 7.6 times as antigenic as theophylline and hence should not require such prohibitive amounts of antibody as required by compound VI to inhibit the electrochemical

reaction. It should be noted, however, that BOTH compound III and compound VI acted as mediators for the glucose oxidase reaction.

An investigation of the change in peak current with increasing compound III concentration was carried out for both the uncoupled and glucose oxidase coupled reactions, the results of which are summarized in the table 7C below

TABLE 7C

| Concentration of Compound III (μM) | $i_p$ coupled (μA) | $i_p$ uncoupled (nA) |
|---|---|---|
| 20 | 0.3 | 9.3 |
| 40 | 0.62 | 25.1 |
| 60 | 0.89 | 93 |
| 80 | 1.13 | 148 |
| 100 | 1.36 | 177 |

A scan rate of 5mV $s^{-1}$ was used for both the coupled and uncoupled experiments.

As can clearly be seen, a linear relationship between peak current and concentration exists for the coupled reaction. However, no such relationship was apparent in

the uncoupled reaction. This is probably due to errors in the estimation of the very small peak currents produced by this reaction, (see attached figure).

(j) Investigation of the effect of theophylline antibodies on the glucose oxidase coupled electrochemistry of compound III

Procedure

A 200ul sample of a 100uM stock solution of compound III, prepared in phosphate buffer pH 7.1 containing 10% (v:v) DMF, was diluted by the addition of either 50μl of phosphate buffer or 50μl of undiluted rabbit antiserum (Immunotech.). From this solution, 150μl were placed in a 0.5ml electrochemical cell containing 250μl buffer and 50μl of a 1M glucose solution.

The d.c. cyclic voltammogram of the uncoupled reaction was then recorded using a scan rate of 5mV $s^{-1}$. Thereafter, 50μl of a 3mg/ml solution of glucose oxidase was added, the solution degassed using argon, and the voltammogram of the enzyme catalysed regeneration of ferrocene from the ferricinium ion recorded. The final concentration of compound III was 24μM.

Further samples were prepared containing an equal

concentration of theophylline and compound III and theophylline at a concentration 10 times that of compound III. In both cases the coupled and uncoupled voltammograms were recorded.

Finally, theophylline (240µM) and compound III (24µM) were incubated with 50µl of undiluted rabbit antiserum for 15 minutes at room temperature prior to a sample being removed and the voltammograms recorded in the absence and presence of glucose oxidase.

Results

The uncoupled cyclic voltammogram of a 24µM solution of compound III showed the expected behaviour of a one-electron redox reagent ($E_{1/2}$ = 195mV, $E_p$ = 60mV, $i_p$ = 25nA). Upon addition of glucose oxidase to the solution, the previously outlined change in the voltammogram occurred. No peaks were observed and a large catalytic current (0.3µA) flowed at oxidizing potentials.

For the case in which compound III was pre-incubated with rabbit antiserum the voltammogram showed no reversible behaviour, with only an anodic peak at 240mV and no peaks at reducing potential. This peak was shown to be due to an unknown component in the antiserum

itself. Upon addition of glucose oxidase, no catalytic current was observed and indeed the voltammogram was identical to that of the uncoupled reaction, indicating that compound III had been completely bound to the antibodies such that it could not take part in electron transfer reactions at the gold electrode and could not act as a mediator for glucose oxidase.

The addition of free theophylline to a solution of compound III at the concentrations previously mentioned had no effect whatsoever on the electrochemical behaviour of the ferrocene-drug conjugate in both the absence and presence of glucose oxidase. When theophylline (240μM) and compound III (24μM) were pre-incubated with rabbit antiserum and the voltammograms recorded it was apparent that compound III was now free to take part in electron transfer reactions at the electrode and could act as a mediator for glucose oxidase. Indeed the catalytic current produced (0.3uA) was identical to that obtained in the absence of both theophylline and antiserum. This is taken as indicative of theophylline binding competitively to the antibodies - thus producing free compound III in solution which is then able to take part in the enzyme coupled reaction, and consequently is a basis for the assay of theophylline in serum.

One form of this assay could alternatively comprise a dry strip in which the theophylline/ferrocene conjugate, the theophylline antibody, glucose oxidase, glucose are placed on the active electrode so that at least the conjugate is separate from the antibody and the glucose oxidase is separate from the glucose (either by spatial location or physical state), and the above constitutes are one aspect of the present invention. When a biological liquid containing an unknown theophylline level is added a competitive binding reaction is set up as described above and the current falls off as the assay progresses for measurement also as described above.

The preferred configuration would be to set up a competitive binding reaction between on the one hand the ferrocene/theophylline to be assayed, for binding sites on the antibodies. To do this the following procedure is effected:-

(a) the unknown amount of theophylline is taken

(b) the conjugate is mixed therewith

(c) the mixture of (a) and (b) is added to the excess glucose (glucose oxidase system in the presence of a sensor electrode e.g. a gold electrode in a two or three electrode system.

(d) the catalytic current is measured

(e) the known amount of antibody is added, to set up a competitive binding reaction as between theophylline and conjugate

(f) the decrease, or rate of decrease, in current is measured.

CLAIMS

1.A method of assay in which (a) at least one specific binding reaction stage between reactive species comprising a ligand and an antiligand is carried out in association with an enzyme/mediator system electrochemically linked to a level of a substrate to which the enzyme is catalytically active so that the binding reaction affects the electrochemical availability of at least one of the components of said system and (b) the effect on the said electrochemical availability is detected or measured to give a signal from which the presence or amount of the said reactive species can be established.

2. A method as claimed in claim 1 in which the specific binding reaction takes place in solution.

3. A method as claimed in claim 1 in which the specific binding reaction takes place at a solid surface.

4. A method as claimed in claim 1 in which an unknown amount of a ligand species to be determined is subjected to a specific binding reaction with a known amount, known to be in excess, of a species antiligand therefor itself chemically linked with the said mediator compound which antiligand species in its unbound state only is

available for charge transfer from the enzyme/substrate reaction, whereby the subsequent electrochemical activity of the excess unbound mediator/antiligand species provides a measure of the amount of ligand species.

5. A method of homogeneous enzyme immunoassay which comprises:

(a) (i) mixing a sample containing ligand to be assayed with a known amount, known to be in excess, of an antiligand whereby some ligand-binding sites on the antiligand are left free;

(ii) mixing with the resultant mixture a known amount, known to be in excess, of the ligand chemically linked with a mediator compound whereby all of the previously free ligand-binding sites are occupied and some ligand-linked mediator remains electrochemically available;

(b) mixing an enzyme and a substrate therefor in a liquid mixture;

(c) contacting the enzyme/substrate mixture with the mixture from stage (a); and

(d) contacting the resulting mixture with a sensor electrode;

whereby the charge transferred to the electrode is dependent upon the amount of available unbound ligand-linked mediator and thus permits derivation of the amount of the original ligand.

6.A method as claimed in claim 5 in which the mediator is a ferrocene.

7. A method as claimed in claim 6 in which the ferrocene is a carboxyl ferrocene.

8. A method as claimed in claim 4, 5 or 6 in which the substrate/enzyme system is chosen from glucose/glucose oxidase and glucose/glucose dehydrogenase.

9 A method as claimed in claim 1 in which (i) (a) an unknown amount of ligand species X to be assayed and (b) an amount of a species X = E, in which X is chemically linked with an enzyme E without destroying the enzymatic activity thereof, are mixed in solution (ii) the mixture is contacted with a substrate S for the enzyme and a mediator compound M in the presence of a sensor electrode to which a measured charge is thereby transferred in dependence on the enzymatically catalysed

reaction (iii) antiligand A is contacted with the solution to set up competitive equilibrium binding reactions of the general nature of reactions I and II

A + X + X = E    I. A - X = E + X

II. A - X + X = E

whereby part of the enzymatically active species X = E is converted to the enzymatically inactive species A - X = E thereby altering the extent of reaction and the measured charge at the electrode and (iv) there is derived from the decrease in charge or rate of decrease in charge a measure of the concentration of analyte X.

10. A method as claimed in claim 1 in which (i) (a) an unknown amount of ligand species X to be assayed and (b) an amount of a species X = M in which X is chemically linked with a mediator M with destroying the mediator activity thereof are mixed in solution (ii) the mixture is contacted with a substrate S and an enzyme E catalytically active thereon in the presence of a sensor electrode to which a charge is thereby transferred for measurement in dependence upon the enzymatically catalysed resction (iii) antiligand A is contacted with the solution to set up competitive equilibrium binding reactions I and II

$$A + X + (X = M) \rightleftharpoons \text{I. } (A - X = M) + X$$

$$\rightleftharpoons \text{II. } (A - X) + (X = M)$$

whereby part of the species X = M is converted to the inactive species A - X = M thereby altering the extent of reaction and the charge measured at the electrode and (iv) there is derived from the decrease in charge or rate of decrease in charge a measurement of the concentration of ligand analyte X.

11. A method of immunoassay for a species, which comprises:

(a) covalently linking an analyte X and an enzyme E to give an enzymatically active species X-E;

(b) mixing with the covalently linked species X-E a suitable reactive ligand A to form an enzymatically inactive species A-X-E with its specific binding sites fully occupied;

(c) contacting the species A-X-E with the species X to be assayed, so as to set up a competitive specific binding reaction with species X and achieve the

equilibrium

$$A\text{-}X\text{-}E + X \rightleftharpoons A\text{-}X + X\text{-}E$$

so that the amount of species X-E is a measure of the amount of species X to be assayed; and contacting the mixture with a substrate for X=E, and transferring charge from the consequent enzymatically catalysed reaction to a sensor electrode by means of a mediator compound to give a measure of the amount of enzymatically active species X=E present, from which the amount of species X can be derived.

12.A method as claimed in claim 9 in which the mediator is a ferrocene.

13. A method as claimed in claim 12 in which the ferrocene is a carboxyferrocene.

14. A method as claimed in claim 9, 10 or 11 in which the substrate/enzyme system is chosen from a glucose/glucose oxidase and a glucose/glucose dehydrogenase.

15. A method as claimed in claim 1 in which at least one of the mediator and enzyme is chemically linked to a nucleic acid probe sequence whereby specific binding of the probe sequence to the target sequence in a

single-strand nucleic acid material to be investigated affects the electrochemical availability of the chemically linked species as detected by a sensor electrode in presence of the enzyme substrate, whereby the presence of the target sequence can be detected

16. A method of detecting a target sequence in a nucleic acid material which comprises:

(a) providing a single strand nucleic acid material to be investigated for a given target sequence;

(b)selecting a probe material with a sequence of nucleic acids complementary to the target sequence;

(c) choosing a procedure from among

(i) chemically linking the probe with an enzyme and adding the enzyme-linked probe to a solution containing both a substrate for the enzyme and a mediator,

(ii) chemically linking the probe with a mediator and adding the mediator-linked probe to a solution containing both a substrate and an enzyme for the said substrate;

(iii) chemically linking the probe with a mediator

enzyme combination and adding the so-modified probe to a solution containing a substrate for the enzyme;

(d) contacting the solution containing the chemically linked probe sequence with a sensor electrode whereby charge is transferred by the mediator to the electrode from the enzyme-catalysed substrate reaction; and
(e) contacting the solution with the single stranded material,

whereby alteration in the amount of charge transferred is an indication of a specific binding reaction between the probe and target affecting the availability of enzyme, mediator or combination.

17. A method as claimed in claim 15 wherein the mediator is linked indirectly to the probe sequence by a linker group and a material reactive to the linker group is present on the electrode, whereby the whole complex is present on the electrode and binds to the probe sequence to cause an alteration in electrode current.

18. A method as claimed in claim 15, 16 or 17 in which the mediator is a ferrocene.

19. A method as claimed in claim 15, 16 or 17 in which the mediator is a carboxyferrocene

20. A method as claimed in claim 15, 16 or 17 in which the substrate/enzyme system is chosen from a glucose/glucose oxidase and a glucose/glucose dehydrogenase system.

21. A method as claimed in claim 1 which comprises immobilising a ligand to be assayed on a suitable surface, thereafter carrying out a specific binding reaction with excess of a suitable antiligand itself chemically linked to an enzyme, prior to remixing the excess and adding a substrate for the immobilised enzyme; and contacting with a mediator and a sensor electrode, so that the charge transferred to the electrode is proportional to the amount of enzyme present.

22. A method of heterogeneous enzyme-linked immunoassay comprising:

(a) immobilising at a suitable surface a ligand to be assayed;

(b) contacting with the ligand excess of an enzymatically active species consisting of an antiligand chemically linked with an enzyme to bring about a specific binding reaction with the immobilised ligand

and give an immobilised enzymatically active species in an amount corresponding to that of the ligand to be assayed;

(c) removing excess non-immobilised chemically-linked enzyme/antiligand, and

(d) contacting the immobilised enzyme with a substrate therefor and with charge-transferring mediator compound whereby an electrode in contact therewith signals a charge corresponding to the amount of immobilised enzyme, from which the original amount of ligand to be assayed may be derived.

23. A method as claimed in claim 22 in which the mediator is a ferrocene.

24. A method as claimed in claim 23 in which the mediator is a carboxyferrocene.

25. A method as claimed in claim 21, 22 or 23 in which the substrate/enzyme is chosen from glucose/glucose oxidase and glucose/glucose dehydrogenase.

26. A method as claimed in claim 1 in which the specific binding reaction takes place at the surface of a sensor electrode to block or alter the characteristics of the

surface at least in part whereby the existence or amount of a decrease in detected electrical charge is a measure of the existence or extent of the specific binding reaction.

27. A method of immunoassay comprising the steps of:

(a) taking a liquid medium containing a ligand to be assayed;
(b) adding thereto a known amount of a substrate capable of undergoing enzyme-catalysed reaction with an enzyme;

(c) contacting the liquid medium with a sensor electrode comprising at its surface a combination of (i) antiligand capable of undergoing a specific binding reaction with the ligand to be assayed (ii) the said enzyme and (iii) a mediator compound to transfer charge from the enzyme to the electrode when the enzyme is catalytically active, so as to provide a signal; and

(d) comparing the signal with a signal received in the absence of the ligand, and deriving the amount of ligand present as a function of blockage or conformational change at the electrode surface caused by the specific binding reaction.

28. A method as claimed in claim 26 in which the

mediator is a ferrocene.

29. A method as claimed in claim 27 in which the mediator is a carboxyferrocene.

30. A method as claimed in claim 26, 27 or 28 in which the substrate/enzyme system is chosen from glucose/glucose oxidase and glucose/glucose dehydrogenase.

31. A method as claimed in claim 1, 2 or 3 in which the enzyme and mediator are chemically linked together.

32. A method for determining the presence of a selected ligand species present in a mixture of compounds, comprising

reacting the ligand species with an anti-ligand to form a ligand/anti-ligand complex,

performing an enzyme catalyzed reaction whose rate is representative of the amount of said complex formed, and

sensing said enzyme catalyzed rate electrochemically by exposing a mixture comprising said enzyme and an electron-transfer mediator to an electrode having an

electrically conductive surface, whereby said electron-transfer mediator is capable of transferring electrons between said electrode surface and said enzyme at a rate representative of said enzyme-catalyzed reaction rate.

33. A method as claimed in claim 10 in which the chemically linked mediator-analyte conjugate is Fc - $CH_2$-theophylline

34. An electrode configuration supporting at its surface (a) a conjugate between a ligand species and a mediator, (b) an antiligand therefor (c) an enzyme substrate and (d) an enzyme catalytically active on the substrate, wherein at least the conjugate is separate from the antiligand and the enzyme is separate from its substrate either by spatial location or physical state.

35. A method of assay in which an electrode as claimed in claim 34 is contacted with a medium containing an unknown amount of the ligand species to be assayed, and the signal or rate of change of signal received fromthe electrode is observed as a measure of the said unknown amount .

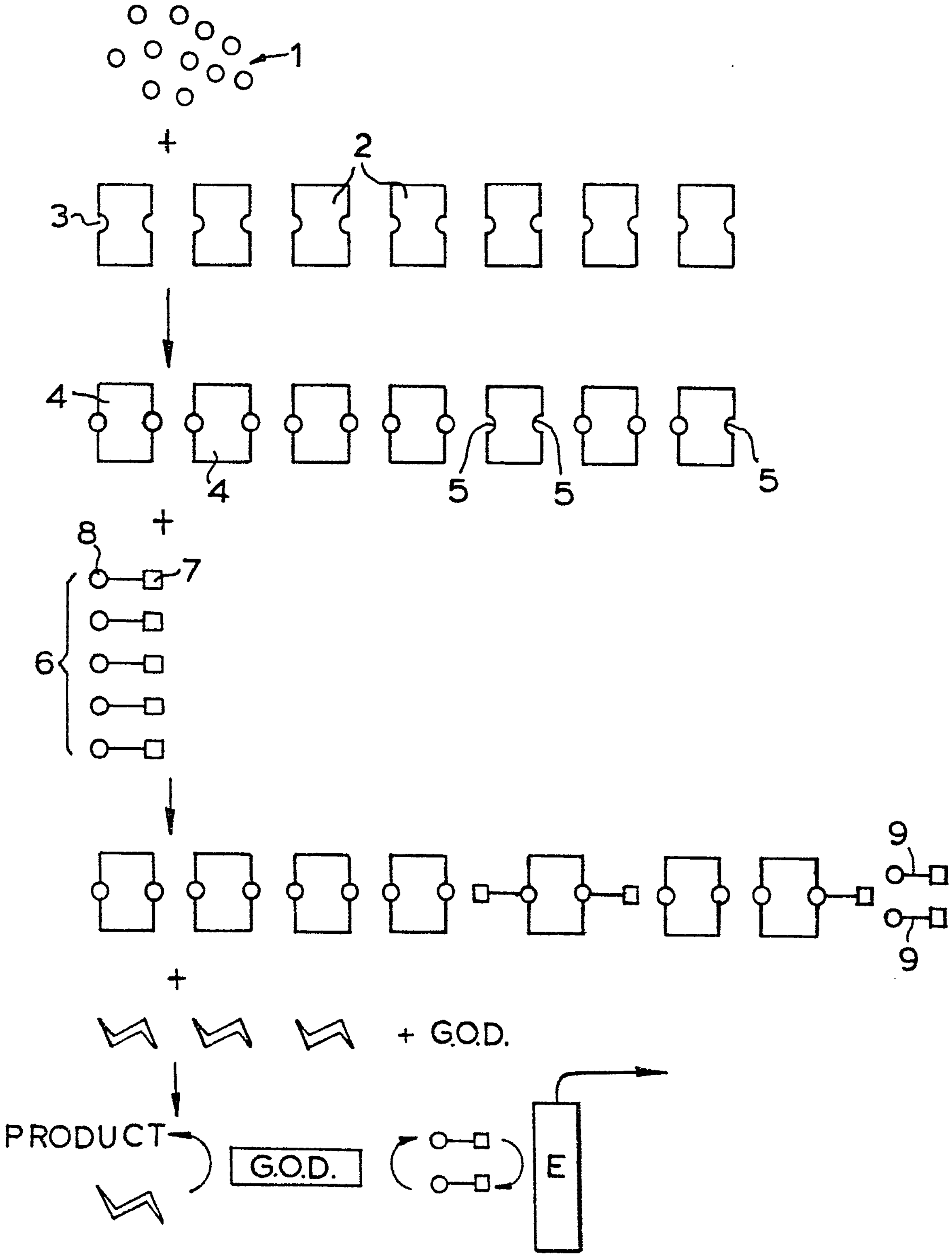


FIG. 1.

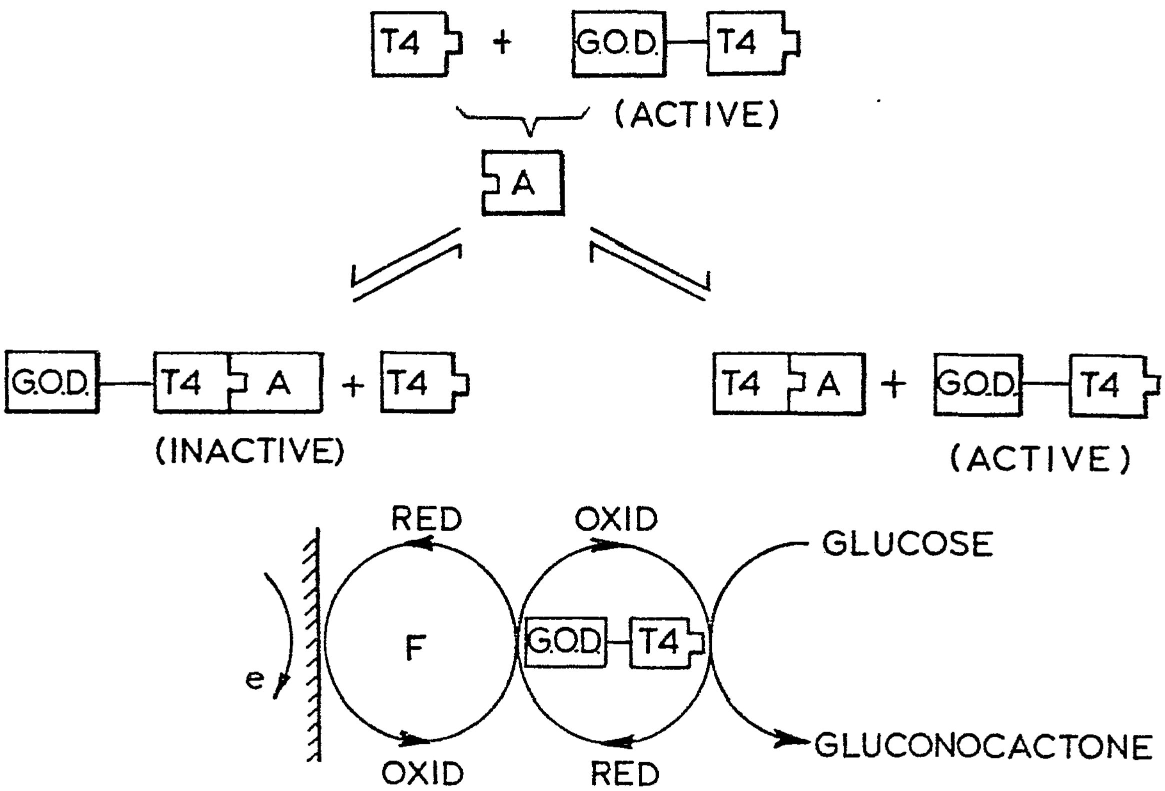
T4
+
G.O.D.
T4
(ACTIVE)
A
G.O.D.
T4
A
+
T4
(INACTIVE)
T4
A
+
G.O.D.
T4
(ACTIVE)
RED
OXID
GLUCOSE
e
F
G.O.D.
T4
OXID
RED
GLUCONOCACTONE

FIG. 2.

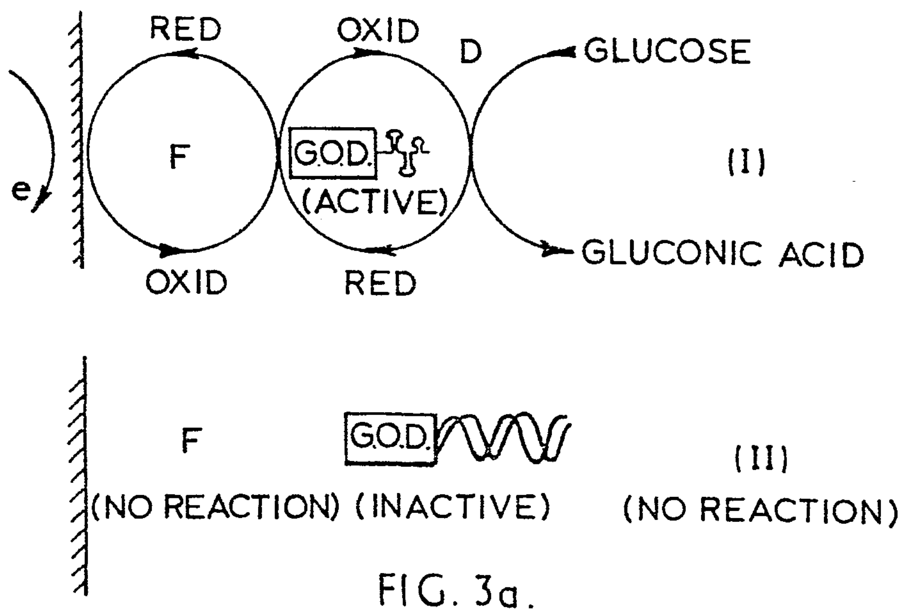
RED
OXID
D
GLUCOSE
e
F
G.O.D.
(ACTIVE)
(I)
OXID
RED
GLUCONIC ACID
F
G.O.D.
(II)
(NO REACTION)
(INACTIVE)
(NO REACTION)

FIG. 3a.

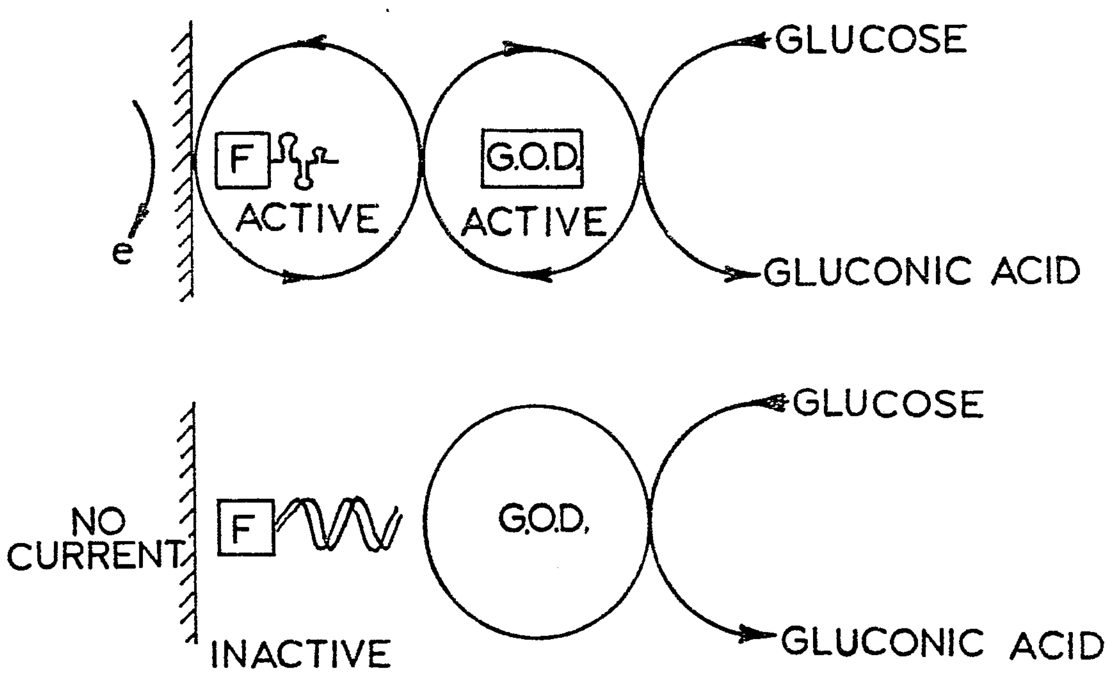
GLUCOSE
F
ACTIVE
G.O.D.
ACTIVE
e
GLUCONIC ACID
GLUCOSE
NO
CURRENT
F
G.O.D.
INACTIVE
GLUCONIC ACID

FIG. 3b.

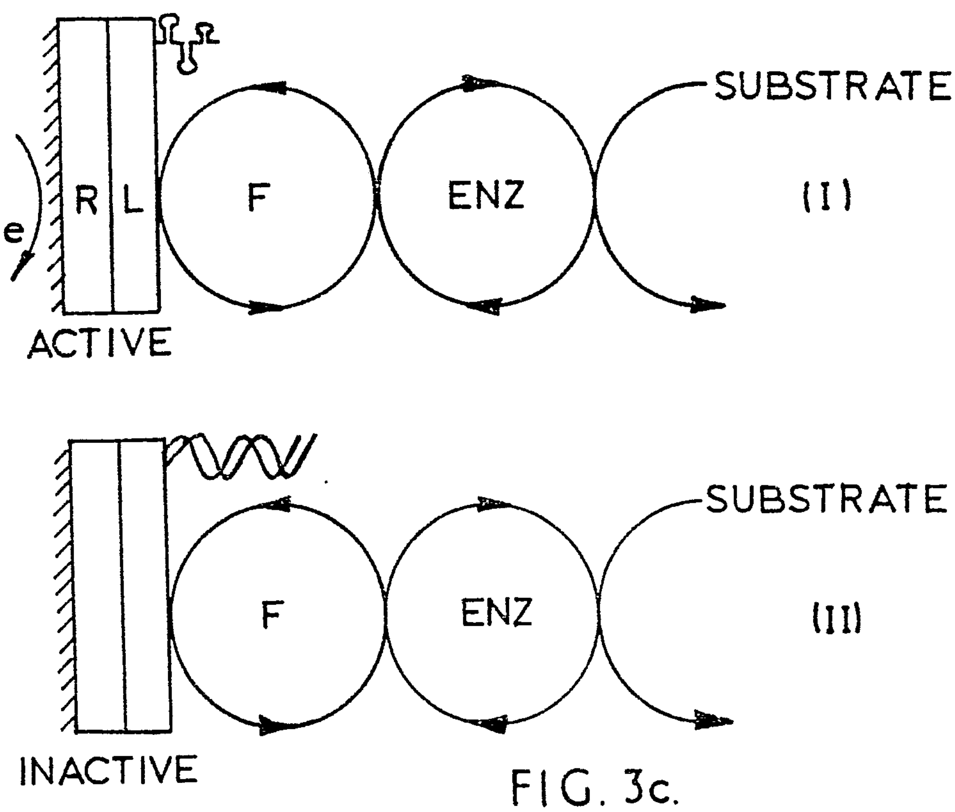
SUBSTRATE
R
L
F
ENZ
(I)
e
ACTIVE
SUBSTRATE
F
ENZ
(II)
INACTIVE

FIG. 3c.

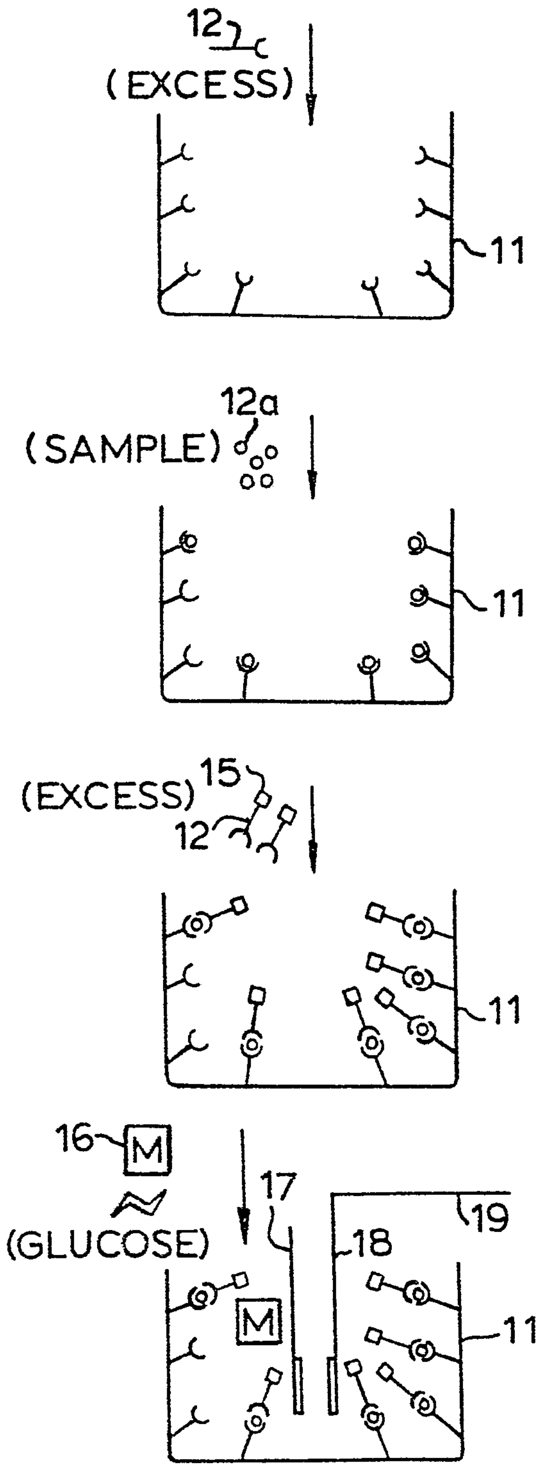

12
(EXCESS)
11
12a
(SAMPLE)
11
(EXCESS)
15
12
11
16
M
17
19
(GLUCOSE)
18
M
11


FIG. 4.

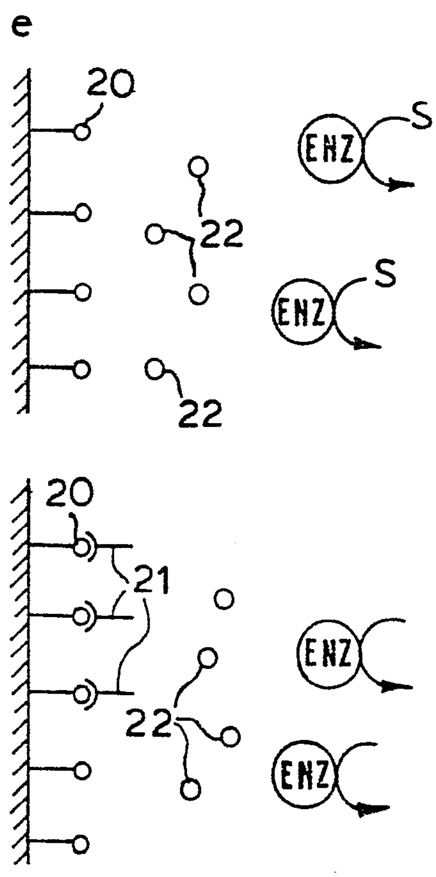

e
20
S
ENZ
22
S
ENZ
22
20
21
ENZ
22
ENZ


FIG. 5.

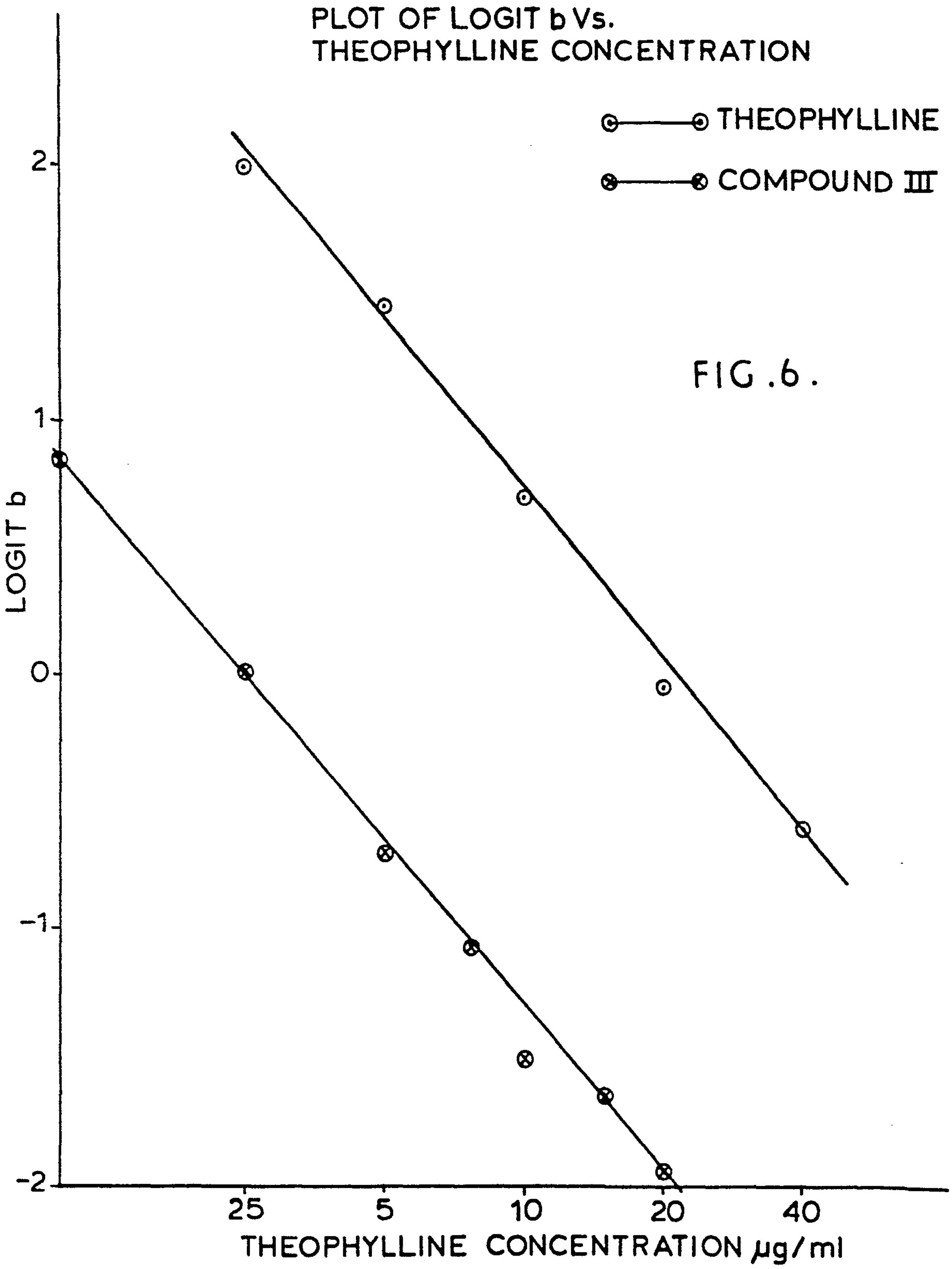
PLOT OF LOGIT b Vs.
THEOPHYLLINE CONCENTRATION
THEOPHYLLINE
COMPOUND III
2
1
0
-1
-2
LOGIT b
25
5
10
20
40
THEOPHYLLINE CONCENTRATION µg/ml

FIG.6.